# EUROPEAN PATENT SPECIFICATION

(11) **EP 3 700 602 B1**
(45) Date of publication and mention of the grant of the patent: **16.10.2024**
(21) Application number: 18811964.8
(22) Date of filing: 26.10.2018
(51) Int. Cl.: A61M 1/00, A61M 3/02, A61B 5/02

(54) **MONITORING FLUSHING LIQUID DURING AN OPERATION**
ÜBERWACHUNG VON SPÜLFLÜSSIGKEIT WÄHREND EINER OPERATION
SURVEILLANCE DE LIQUIDE DE RINÇAGE PENDANT UNE OPÉRATION

(30) Priority: 26.10.2017 US 201762577519 P; 24.10.2018 US 201816169603
(43) Date of publication of application: 02.09.2020
(60) Divisional application: 24199136.3
(73) Proprietor: Edwards Lifesciences Corporation, Irvine, CA 92614 (US)
(72) Inventor: LI, Peiyuan, 1019 HD Amsterdam (NL); VAN DER SAR, Geertruida, Lucretia, Irvine CA 92614 (US)
(74) Representative: Eisenführ Speiser
(86) International application number: PCT/US2018/057659
(87) International publication number: WO 2019/084368

(56) References cited:
- WO-A1-2017/066783
- WO-A1-2017/066783
- WO-A2-2012/097141
- WO-A2-2012/097141
- US-A- 5 709 670
- US-A1- 2013 218 106
- US-A1- 2016 331 888

## Description

### BACKGROUND

### Field

The present disclosure relates generally to monitoring flushing liquid during an operation, and more particularly to systems, apparatuses, and methods for determining a volume of flushing liquid delivered to a patient.

### Description of Related Art

During surgery in an operating room, it is beneficial for doctors to monitor the amount of fluid lost from and given to a patient to maintain a fluid balance. One aspect of this process is to monitor the amount of blood that is lost. To do so, doctors typically use equipment to direct the lost blood into a container that is used to estimate the amount of blood loss. At the same time, doctors typically use flushing liquids to clean and/or clear the surgery location, which is also directed into the same container. As result, it may be difficult to accurately determine the amount of blood lost by the patient. One way of determining the amount of flushing liquid that has been used is determine the amount of liquid left in the flushing IV bag. However, this method lacks accuracy as it is merely an estimation.

Such lack of accuracy in determining fluid loss may negatively impact the ability of healthcare professionals to treat the patient and/or to confidently assess the condition of the patient.

Physiological indicators such as stroke volume (SV), cardiac output (CO), end-diastolic volume, ejection fraction, stroke volume variation (SW), pulse pressure variation (PPV), systolic pressure variations (SPV), and plethysmographic variability index (PVI) have been found useful in the assessment of patient status and conditions, particularly in medium to high risk patients undergoing surgery or in the intensive care unit. Many clinicians utilize these parameters to determine how much fluid [or medications] to deliver to their patients, and clinical studies have shown that when used in conjunction with a treatment protocol, these types of parameters guide treatment in a way that improves patient outcomes. For instance, clinicians may give a "fluid challenge," e.g., give the patient a bolus of fluid (ranging from about 200 mL to about 1 L), and observe the corresponding change in the SV. A large change in SV indicates that the patient is "fluid responsive" and may benefit from more fluid; a small change in SV may indicate that the patient is not responsive to fluid and is therefore unlikely to benefit from additional fluid. In spite of their utility, the application of these parameters and protocols has been limited due at least in part to difficulties in tracking the amount of fluid or medication corresponding to the physiological change in the parameters. Highly skilled and/or well-trained clinicians can manually keep track of the timing/rate/amount of the fluids/medication and match it to the corresponding change in physiological parameters, however, the process is tedious and prone to error. Thus, only the most highly skilled, well trained, and patient clinicians can maintain a high compliance rate to desired treatment protocols.

Software in conventional monitoring systems has attempted to simplify this process by providing calculations and recommendations. However, typical monitoring systems are not configured or equipped for determining or measuring all the fluids and/or medications that are delivered to the subject (e.g., fluid and/or medication delivered through multiple delivery devices simultaneously, concurrently, or intermittently). While in theory this could be accomplished by a clinician manually entering the relevant information, such a process is time-consuming, tedious, and prone to human error.

While methods of precisely tracking fluid using a conventional fluid infusion pump exist, in practice such equipment and methods have proven sub-optimal for various reasons, including that the information from conventional fluid infusion pumps is not connected to physiological monitors, so the calculations, tracking, and comparisons still require significant manual input. Furthermore, standard fluid infusion pumps generally have a fluid delivery limitation of around 1 L/hour, which may be slower than a desired infusion rate. In addition, there are infusion pumps on the market that limit and/or slow integration with existing, commercially-installed monitor base systems. Moreover, conventional systems are typically not configured or equipped for determining or measuring all the fluid that is being delivered to the subject (e.g., fluid delivered through multiple delivery devices simultaneously, concurrently, or intermittently).

Thus, even if healthcare providers desire implementation of a protocolized approach to fluid and/or medication delivery, they are left with sub-optimal options. Currently, there is no easy method of comparing the amount of fluid and/or medication given a patient and the corresponding change in physiological parameters resulting therefrom to implement a robust protocolized approach to fluid and/or medication delivery optimization.

WO 2017/066783 discloses an interoperative blood loss monitoring system. The system senses the volume or weight of fluid in the sterile bucket and determines any decrease in the volume or weight of fluid in a sterile bucket and determines a decrease in the volume or weight to be an introduction of fluid to the patient. If, however, additional saline is added to the bucket, that addition is not counted in the fluid tracking and the further decrements from that new fluid amount is what is used to track fluid addition to the patient.

WO 2012/097141 describes a system and method for patient-adaptive hemodynamic management. It includes transfusion, volume resuscitation with intravenous fluids, and medications, utilizing monitored hemodynamic parameters including the described dynamic predictors of fluid responsiveness, and including an algorithm capable of adaptation of the function of the device to specific patients.

### SUMMARY

In a first aspect, the present disclosure relates to a subject monitoring system according to claim 1.

The disclosure relates to a system for monitoring an amount of blood lost from a subject. The system includes a flushing liquid device comprising a flushing liquid source, a conduit configured to direct flushing liquid from the flushing liquid source to the subject, and a flow sensor coupled to the conduit, the flow sensor configured to provide flow-related signals corresponding to a flow of the flushing liquid through the conduit. The system also includes a collection container configured to receive effusion liquid from the subject, the effusion liquid including blood and flushing liquid. The system also includes a collection container sensor configured to provide volume-related signals corresponding to a volume of the effusion liquid in the collection container. The system also includes a monitor configured to receive the flow-related signals from the flow sensor, to receive the volume-related signals from the collection container sensor, and to determine a volume of blood in the collection container.

In some embodiments of the first aspect, the flow sensor comprises an ultrasonic transducer. In some embodiments of the first aspect, the flow sensor is a disposable.

In some embodiments of the first aspect, the flushing liquid device further comprises a processor coupled to the flow sensor, the processor configured to execute computer-executable instructions to derive a volume of flushing liquid delivered to the subject based at least in part on the flow-related signals provided by the flow sensor. In further embodiments of the first aspect, the processor is configured to provide flushing liquid volume signals to the monitor.

In some embodiments of the first aspect, the monitor is configured to display the determined volume of blood in the collection container. In some embodiments of the first aspect, the collection container sensor is configured to provide signals indicative of a weight of liquid in the collection container. In some embodiments of the first aspect, the system further includes an effusion pump configured to direct the effusion liquid from the subject to the collection container.

In some embodiments of the first aspect, the flushing device further comprises a pump configured to direct the flushing liquid from the flushing liquid source to the conduit. In some embodiments of the first aspect, the system further includes a controller configured to control the pump to control a flow of the flushing liquid from the flushing liquid source.

In a second aspect, which is not claimed, a method is provided for determining blood lost from a subject during a surgical procedure. The method includes measuring, using a flow sensor, a flow rate of flushing liquid from a flushing device. The method also includes determining a volume of flushing liquid delivered to the subject based at least in part on the measured flow rate. The method also includes measuring a volume of an effusion liquid in a collection container, the effusion liquid including a combination of blood and the flushing liquid. The method also includes calculating a volume of blood in the collection container based at least in part on the measured volume of effusion liquid and the determined volume of flushing liquid delivered to the subject.

In some embodiments of the second aspect, the flow sensor provides flow-related data indicative of a flow rate of the flushing liquid. In some embodiments of the second aspect, the method further includes displaying the calculated volume of blood in the collection container. In some embodiments of the second aspect, measuring the volume of the effusion liquid comprises receiving volume-related signals from a collection container sensor coupled to the collection container. In some embodiments of the second aspect, the method further includes directing the effusion liquid from the subject to the collection container. In some embodiments of the second aspect, the method further includes directing the flushing liquid from a flushing liquid device to the subject. In some embodiments of the second aspect, the method further includes controlling a flow rate of the flushing liquid to the subject.

### BRIEF DESCRIPTON OF THE DRAWINGS

Various embodiments are depicted in the accompanying drawings for illustrative purposes, and should in no way be interpreted as limiting the scope of the inventions which are delimited by the appended set of claims.

In addition, various features of different disclosed embodiments can be combined to form additional embodiments, which are part of this disclosure. Throughout the drawings, reference numbers may be reused to indicate correspondence between reference elements.
FIG. 1A is a schematic flow diagram of an example subject monitoring system.
FIG. 1B is a schematic flow diagram of another subject monitoring system.
FIG. 1C is a schematic flow diagram of another subject monitoring system.
FIG. 1D is a schematic flow diagram of another subject monitoring system.
FIG. 1E is a schematic flow diagram of another subject monitoring system.
FIG. 1F is an example schematic block diagram of the subject monitoring system of FIG. 1A.
FIG. 2 is a block diagram of an example embodiment of system electronics.
FIGS. 3A and 3B are representations illustrating various embodiments of a system configuration for managing a hemodynamic state.
FIGS. 4A and 4B are representations illustrating various embodiments of a system configuration for managing a hemodynamic state.
FIG. 5 is a sectional view of an exemplary solenoid flow controller as disclosed and described herein.
FIG. 6 is a diagram representation of an exemplary weight sensor as disclosed and described herein.
FIG. 7 is a flow chart of an example method as disclosed and described herein.
FIG. 8 is a flow chart of another example method as disclosed and described herein.
FIG. 9 is a flow chart of another example method as disclosed and described herein.
FIG. 10 is a flow chart of another example method as disclosed and described herein.
FIG. 11 is a flow chart of another example method as disclosed and described herein.
FIG. 12 illustrates a block diagram of an example flushing liquid device.
FIG. 13 illustrates a block diagram of an example monitoring system for use with the flushing liquid device of FIG. 12.
FIG. 14 is a flow chart of another example method as disclosed and described herein.

### DETAILED DESCRIPTION

The headings provided herein are for convenience only and do not necessarily affect the scope or meaning of any of the claimed embodiments.

### Overview

During a surgery in an operating room, to maintain fluid balance in patients, doctors monitor the amount of fluid given to the patient as well as the amount of fluid lost from the patient. This process includes monitoring how much blood is lost by the patient. To do so, doctors typically use equipment to suck the lost blood to a collection container. The collection container is used to estimate the amount of blood loss. One complicating factor is that doctors typically use flushing liquid to clean and/or clear the surgical location. This fluid is also typically sucked into the same collection container. Consequently, it is difficult to accurately determine the amount of blood lost by the patient. A typical solution is to track the amount of used flushing liquid by determining the amount of fluid left in the fluid source (e.g., the flushing IV bag). However, this method is an estimation and may lack the desired accuracy. Such a lack of accuracy may negatively impact the treatment of the patient and/or the judgement of the doctors.

Accordingly, to address these and other issues, disclosed herein are flushing devices, systems, and methods that accurately track the amount of flushing liquid delivered to a patient. Devices are disclosed that monitor the usage of flushing liquid during an operation using a flow sensor or flow probe. By accurately determining the amount of used flushing liquid, the amount of blood loss can be calculated accurately as well. This advantageously helps doctors to more accurately assess the status of the patient.

The disclosed devices, systems, and methods use a flow sensor to measure the flow rate of liquid through a conduit. The flow sensor can be clamped on or integrated with a conduit of the flushing device. The flow sensor can be configured to detect the liquid flow speed or flow rate. Using the flow-related data, a processor can employ an algorithm to calculate the amount of flushing liquid used. This information can be displayed and/or provided to a fluid monitoring system. The flushing liquid volume and/or flow rate can be displayed on a simple display on the sensor housing, for example, and/or it can be sent to a hospital information system. With this information, together with the total volume of the collecting container, a monitoring system can accurately determine how much blood is lost from the patient.

In addition, the present disclosure provides systems that include a flow probe that senses fluid flow or mass flow of fluid delivery to a patient. Based at least in part on the sensed fluid or mass flow, the flow probe provides flow-related data (e.g., fluid or mass flow rate) that the system uses to derive a volume of fluid being delivered. The fluid can be delivered from an IV bag, another in-line port, or a combination of the two. Briefly, and as further described below, the disclosed systems provide fluid volume and/or fluid rate for display to a clinician and an informative understanding of what volume of fluid/mass a patient has received. This system finds applicability in determining an amount of fluid delivered, and may be applicable without any flow controlling device or means.

The disclosed systems can include a flow probe, a physiological sensor, and a monitor (and an algorithm) with a graphical user interface (GUI). The flow probe can be configured to sense flow-related information in combination with sensed physiological data from the physiological sensor. In such embodiments, the physiological sensor information can be utilized along with the fluid delivery related data (e.g., provided by the flow probe) to generate a recommendation to the clinician of subsequent administration protocol(s) that can include a targeted flow rate and volume amounts and/or adjustments to the flow rate and/or volume. In certain implementations, no actual mechanism, device, or recommended method of how to administer that fluid is provided. Thus, in such implementations, it is in the discretion of the clinician to control the subsequent administration of fluid based on the recommendation provided by the system.

In some embodiments, the disclosed systems include a flow probe and a physiological sensor that provides physiological data to an algorithm. The algorithm utilizes the flow related information from the flow probe and the physiological sensor data to provide a recommendation to the clinician (e.g., via a GUI) regarding subsequent administration protocol(s) that can include flow rate and volume amounts and/or adjustments. The disclosed systems can further include a flow controller associated with the source of fluid that is manually manipulated to vary fluid delivery rate and, by extension, the volume of fluid delivered. The flow controller can be configured to be controlled by the clinician manually. The actual physical control of fluid administered could be a standard IV roller clamp, for example. Such manual control of fluid delivery may be part of a standard IV tubing set that can be connected and/or adapted to the disclosed systems. Thus, it is in the discretion of the clinician to control the subsequent administration of fluid based on the recommendation provided by the system.

In some embodiments, the disclosed systems include a flow rate sensor and a physiological sensor that provides physiological data to an algorithm. The algorithm utilizes the flow related information and the physiological sensor data to provide subsequent administration protocol(s) that can include flow rate and volume amounts and/or adjustments. The disclosed systems further include a flow controller, associated with the source of fluid that is automatically manipulated by the system to vary fluid delivery rate and, by extension, the volume of fluid delivered. Although the flow controller of the system is configured to be controlled essentially automatically by the algorithm, an ability to override the algorithm and its administration protocol by the clinician is provided.

The physiological sensor of the presently disclosed system includes a hemodynamic sensor, such as the FLOTRAC^{®} sensor, configured to provide information capable of being transformed into one or more forms of heart output data. In some embodiments, an oximetry device can be used as the hemodynamic sensor. In certain embodiments, the oximetry device is a finger cuff device that is integrated with the system, the system electronics, and/or the monitor or algorithm. The disclosed systems can utilize an algorithm for determining how the patient responds to certain administered fluid volumes and rates of fluid administration. Based at least in part on the correlated sensed data from the flow probe and the data from the hemodynamic sensor to that of the patient response, the algorithm can provide information to the clinician regarding subsequent bolus administration and/or control (e.g., through the monitor) the amount and/or rate of fluid delivered to the patient by controlling the flow controller.

The terms "subject" and "patient" are used interchangeably herein and relate to mammals, inclusive of warm-blooded animals (domesticated and non-domesticated animals), and humans. The terms "clinician" and "healthcare provider" are used interchangeably herein.

The phrase "vascular access device" as used herein relates to any device that is in communication (or contact) with the vascular system of a subject. Vascular access devices include but are not limited to catheters, shunts, blood withdrawal devices, connectors, fluid couplers, valves, tubing.

The term "sensor" as used herein relates to a device, component, or region of a device capable of detecting and/or quantifying and/or qualifying a physiological parameter of a subject. The phrase "system" as used herein relates to a device, or combination of devices operating at least in part in a cooperative manner, that is inclusive of the "sensor." Sensors generally include those that continually measure the physiological parameter without user initiation and/or interaction ("continuous sensing device" or "continuous sensor"). Continuous sensors include devices and monitoring processes wherein data gaps can and/or do exist, for example, when a continuous pressure sensor is temporarily not providing data, monitoring, or detecting. In preferred aspects, sensor or continuous sensing device relates to a device, component, or region of a device capable of detecting and/or quantifying and/or qualifying a physiological hemodynamic parameter of a subject.

The phrases "physiological data," "physiological parameter," and/or "hemodynamic parameter" include without limitation, parameters directly or indirectly related to providing or calculating stroke volume (SV), cardiac output (CO), end-diastolic volume, ejection fraction, stroke volume variation (SW), pulse pressure variation (PPV), systolic pressure variations (SPV), extravascular lung water index (ELWI), pulmonary vascular permeability index (PVPI), global end-diastolic index (GEDI), global ejection fraction (GEF), systolic volume index (SVI), arterial blood pressure (ABP), cardiac index (CI), systemic vascular resistance index (SVRI), peripheral resistance (PR), central venous saturation (ScvO2), and plethysmographic variability index (PVI). Hemodynamic parameters are inclusive of the absolute value of such parameters, a percentage change or variation in the parameters since an event was recorded, and an absolute percentage change within a previous time segment.

The term or phrase "coupling" and "operatively coupling" as used herein relate to a joining or linking together of two or more things, such as two parts of a device or two devices, such that the things can function together. In one example, two devices can be operatively coupled by tubing, such that fluid can flow from one device to another. Coupling does not imply a physical connection. For example, a transmitter and a receiver can be operatively coupled by radio frequency (RF) transmission/communication.

The phrases "electronic connection," "electrical connection," "electrical contact" as used herein relate to any connection between two electrical conductors known to those in the art. In one embodiment, electrodes are in electrical connection with (e.g., electrically connected to) the electronic circuitry of a device.

The term and phrase "electronics" and "system electronics" as used herein relate to electronics operatively coupled to the sensor and configured to measure, process, receive, and/or transmit data associated with a sensor, and/or electronics configured to communicate with a flow controller and to control/monitor fluid metering by the flow controller.

The phrase "fluid communication" as used herein relates to two or more components (e.g., things such as parts of a body or parts of a device) functionally linked such that fluid can move from one component to another without limit to or implication of directionality unless stated.

The phrases "operatively connected," "operatively linked," "operably connected," and "operably linked" as used herein relate to one or more components linked to one or more other components, such that a function is enabled. The terms can refer to a mechanical connection, an electrical connection, or any connection that allows transmission of signals between the components. For example, one or more transducers can be used to detect pressure and to convert that information into a signal; the signal can then be transmitted to a circuit. In such an example, the transducer is "operably linked" to the electronic circuitry. The terms "operatively connected," "operatively linked," "operably connected," and "operably linked" include wired and wireless connections.

The term "monitor" as used herein as a noun, refers to a device configured to observe, record, oversee, detect, supervise, regulate, receive, and/or transmit one or more signals, operations or conditions over a fixed, intermittent, or continuous period of time, for example, signals from a flow probe or hemodynamic sensor. The monitor can include a display for presenting data or other information. The monitor can include one or more processors or processing modules.

The term "display" as used herein as a noun, refers to a device configured to provide a visual representation of data (e.g., text and/or graphics and/or symbols) or any other information from a processor, computer, or monitor.

The term and phrase "processor" or "processing module," as used herein relates to components designed to perform arithmetic or logic operations using logic circuitry that responds to and processes basic instructions, for example, instructions that drive a computer and/or perform calculations of numbers or their representation (e.g., binary numbers).

The terms "substantial" and "substantially" as used herein relate to a sufficient amount that provides a desired function. For example, an amount greater than 50 percent, an amount greater than 60 percent, an amount greater than 70 percent, an amount greater than 80 percent, or an amount greater than 90 percent.

Embodiments of the disclosure include closed-loop systems and/or partial closed-loop systems for fluid management that establish communication between a monitor and a source of liquid infusate. The present systems, apparatus, apparatuses, and methods can provide assisted automated delivery of fluid. Assisted automated delivery of fluid, or assisted fluid delivery, can be based on a determination of delivered fluid volume. In some aspects, assisted automated delivery of fluid, or assisted fluid delivery, can be based on determined delivered fluid volume in combination with optimization of physiological hemodynamic parameters. The present systems can be configured to determine total fluid delivered by all sources of infusion, e.g., whether or not delivered by an infusion pump and/or IV bag or other means of fluid administration. The present systems provide for a reduction in the burden placed on a clinician, provide for standardize care, and/or improve or optimize clinical outcomes.

The present apparatuses, systems and methods overcome significant limitations of conventional systems. The present system can be configured to operate using an infusion rate obtained by gravity assist. This can include, for example, a flow rate of about 6 L/hour, e.g., an infusion rate corresponding to a 100 mL bolus delivered in 1 minute. The present system can be also configured for use with a separate fluid delivery device with an infusion rate of about 6 L/hour, 5 L/hour, 4 L/hour, 3 L/hour, or 2 L/hour. The disclosed systems can be readily interchangeable and integrated with previously installed base systems (e.g., agnostic to any infusion pump or other fluid delivery method mechanism). In addition, the acquisition of total fluid volumes of all fluid delivered to the subject are provided, regardless of the mode of infusion (e.g., pump or IV-bag). Moreover, the disclosed systems provide for methods of obtaining and exploiting hemodynamic parameter related infusion events, among other physiological parameters. In some aspects, the disclosed systems provide for methods of obtaining and exploiting hemodynamic parameter related infusion events, among other physiological parameters occurring as a result of an infusion event at an infusion rate of 2 L/hour to about 6 L/hour.

In some aspects, the disclosed systems are devoid of any primary infusion means not otherwise capable of infusing fluid to a subject at a rate of greater than 2 L/hour, such as for example, conventional infusion pumps. To be clear, such conventional infusion pumps may be used in combination with the presently disclosed infusion source, for example, to administer medicament to the subject.

Hemodynamic parameter data obtained by the hemodynamic sensor includes, for example, blood pressure parameters and blood pressure waveform information either in analog or digital form. From such blood pressure parameters, stroke volume variation (SW), pulse pressure variation (PPV), systolic pressure variation (SPV), and plethysmographic variability index (PVI) can be calculated or derived and displayed.

The disclosed systems include programming, for example, in the form of one or more algorithms, configured to diagnose a condition of the subject using the combination of HD-sensor data and flow probe data and corresponding subject responsiveness to infusion events. The systems can be configured to intermittently or continuously determine at least some hemodynamic information (e.g., stroke volume (SV), cardiac output (CO), end-diastolic volume, ejection fraction, stroke volume variation (SW), pulse pressure variation (PPV), systolic pressure variations (SPV), plethysmographic variability index (PVI)). For example, the system can be configured to make such determinations responsive to the HD-sensor sending hemodynamic data and/or the flow probe sending time-related mass flow volume or rate data to the system electronics.

Thus, in some embodiments, the disclosed systems include a hemodynamic sensor (e.g., a FLOTRAC^{™} sensor and/or a CLEARSIGHT^{™} sensor provided by Edwards Lifesciences located in Irvine California) that provides one or more hemodynamic parameter dependent signals or data of a subject to a monitor having an algorithm that utilizes the hemodynamic parameter data, and a flow probe that measures an amount of fluid infused into the subject and an algorithm that utilizes the flow-related data. The algorithms provide recommendations to a health care provider based on the received hemodynamic parameter data and the received flow probe data. The healthcare provider can be presented with a recommendation to infuse fluid, to alter an infusion rate, or to terminate infusion to the subject to adjust or otherwise manipulate one or more hemodynamic parameters. The recommendation can be based on a combination of the flow probe data and the hemodynamic parameter data.

In some embodiments, the disclosed systems include a hemodynamic sensor and a flow probe. In such embodiments, the system provides one or more hemodynamic parameter dependent signals or data of a subject to an algorithm that utilizes the hemodynamic parameter data in combination with a flow probe that measures an amount or rate of fluid infused into the subject and provides one or more administration-related signals or data of the infusion to the algorithm. The algorithm is configured to provide recommendations to a health care provider to infuse or not to infuse fluid to the subject based on the received hemodynamic parameter data and the received flow probe data. The healthcare provider can be presented with a recommendation as to whether or not to accept the prompts relating to recommended administration protocols determined by the algorithms (e.g., the prompts can include recommendations to infuse fluid, to alter an infusion rate, or to terminate infusion to the subject). Upon accepting the recommendation, the system provides one or more signals to a flow controller to adjust or to otherwise manipulate the infusion rate and to manage one or more hemodynamic parameters based on a combination of the flow probe data and the hemodynamic parameter data.

In some embodiments, the disclosed systems include a hemodynamic sensor and a flow probe. The hemodynamic sensor provides one or more hemodynamic parameter dependent signals or data to a monitor having an algorithm that utilizes the hemodynamic parameter data. The flow probe is coupled to a gravity-feed IV bag and measures an amount of fluid infused into the subject and/or provides mass flow information to the algorithm. The algorithm can be configured to provide recommendations to a health care provider based on the combination of the received hemodynamic parameter data and the received flow probe data. The healthcare provider can then be presented with a recommendation to infuse fluid, to alter an infusion rate, or to terminate infusion to the subject to adjust or to otherwise manipulate one or more hemodynamic parameters based on a combination of the flow probe data and the hemodynamic parameter data. In some aspects, the system is configured to operate with an IV bag capable of infusing fluid into the subject using gravity-assistance. In various aspects, the system includes an infusion source capable of infusing fluid into the subject using gravity assistance and controlling the fluid flow rate within a range that includes about 2 L/hour to about 6 L/hour.

In some embodiments, the disclosed systems include a hemodynamic sensor and a flow probe. The hemodynamic sensor provides one or more hemodynamic parameter dependent signals or data corresponding to a subject to an algorithm that utilizes the hemodynamic parameter data and provides recommendations to a health care provider of whether or not to infuse fluid to the subject based on the received hemodynamic parameter data. The flow probe is in communication with a monitor and measures an amount or rate of fluid infused into the subject from at least one infusion source and provides that information to the algorithm.

In some embodiments, the disclosed systems further include a flow controller providing control of the at least one infusion source in fluid communication with the subject.

In some embodiments, the disclosed systems include a gravity feed IV bag as the primary source of infusate of the system. The gravity feed IV bag, in some aspects, can be the only source of fluid infused into the subject. In various implementations, the disclosed systems include a gravity assisted IV bag as the primary source of infusate in combination with one or more secondary sources of infusate. In certain implementations, the disclosed systems include a second infusion source capable of infusing fluid into the subject. The second infusion source can be a gravity assisted source of fluid or can be an infusion pump. In some embodiments, the second infusion source provides for a fluid flow rate different from the first infusion source. For example, the second infusion source provides a fluid flow rate of about 2 L/hour to about 6 L/hour, whereas all other sources of infusion into the subject are at a rate less than about 2 L/hour, less than about 1 L/hour, or less than about 0.5 L/hour.

In some embodiments, the IV bag can be initially pressurized and this pressure can be independently obtained, monitored, and/or maintained. One or more signals representing the pressure of the IV bag can be sent to the algorithm and/or manipulated by the algorithm to provide a constant head pressure of the infusion fluid over one or more intervals of time. In certain implementations, the head pressure of the infusion fluid can be varied over one or more intervals of time.

In certain embodiments, the IV bag can be configured such that its weight is dynamically, intermittently, or continuously monitored. This information can be used to determine mass flow data. The mass flow data may be transmitted to the monitor for incorporation into the algorithm and/or for presentation to the user. This can be done independently or as a backup or redundant system to the flow probe sensor and associated electronics.

As described herein, embodiments of the flow probe can provide dynamic, continuous, intermittent, or on-demand data in the form of analog or digital signals for use by the algorithm or the healthcare provider. The flow probe can be cooperatively engaged with a flow controller and controlled by the algorithm. For example, the flow probe and the flow controller can be used in an open-loop or closed-loop feedback system to control fluid flow based at least in part on measured flow-related data.

The flow probe data can be used to present on the monitor or another display flow-related data via numerical, textual, or pictorial information. This can be displayed or presented in addition to hemodynamic data. The information can include a dynamic mass flow rate and/or a mass flow rate history. The information can also include one or more recommendations as to subsequent administrations.

In some embodiments, where the system lacks any coupled flow controlling device, the healthcare provider can manually control and/or adjust the flow rate including terminating infusion. Manually controlling the flow rate by the healthcare provider can be accomplished by manipulating an independent mechanical device cooperatively engaged with the source of infusate. In certain embodiments, where the system includes an operatively coupled flow controlling device, the healthcare provider can respond to one or more prompts on the monitor or display to facilitate sending one or more electronic signals to the flow controller.

The flow probe and the flow controller can be independently or concurrently controlled by the algorithm. For example, the flow controller can be set to automatically cease infusion upon reaching a threshold infusion rate determined by the flow probe, or the flow probe can detect a disruption in the total mass flow of infusate which can signal an alarm or other indicia on the monitor or display and/or automatically terminate infusion via the flow controller. The algorithm can use existing mass flow rate history in combination with a hemodynamic parameter history to provide predictive management of future infusion events or the lack thereof.

The flow controller can be configured to stop, start, and/or vary the flow rate of the infusate along the flow path between the source of infusion fluid and the subject. The flow controller can be a valve and/or solenoid actuated. In some embodiments, the flow controller can be a mechanical pinch valve operated by a healthcare provider. The flow controller can be independently or concurrently controlled by the algorithm. For example, the flow controller can be set to automatically cease infusion upon reaching a threshold infusion rate determined by the flow probe, or the healthcare provider can intervene and/or override algorithmic control.

In various embodiments, the disclosed systems and methods are configured to find a plateau and/or stable window of data in response to the hemodynamic parameter sensor data, flow probe data, and subject status. In certain embodiments, if the system cannot reach the plateau and/or stable window of data, the system can be configured to "fail-safe." In general, the term "fail-safe" includes modifying the system processing and/or display of data in some manner responsive to a detected error, or unexpected condition, and thereby avoids processing of potentially inaccurate or clinically irrelevant hemodynamic parameter value and changing infusion parameters. In some embodiments, the disclosed systems and methods are configured to process a flow probe signal corresponding to a subject condition to determine whether the flow probe signal corresponds to an infusion rate or amount that is within a predetermined or expected subject condition range or change; if the signal falls outside the expected or predetermined range, the system is configured to fail-safe.

The following description illustrate some example embodiments in detail. Those of skill in the art will recognize that there are numerous variations and modifications of the present disclosure that are encompassed by its scope. Accordingly, the description of certain embodiments should not be deemed to limit the scope of the disclosure.

### Example Subject Monitoring Systems

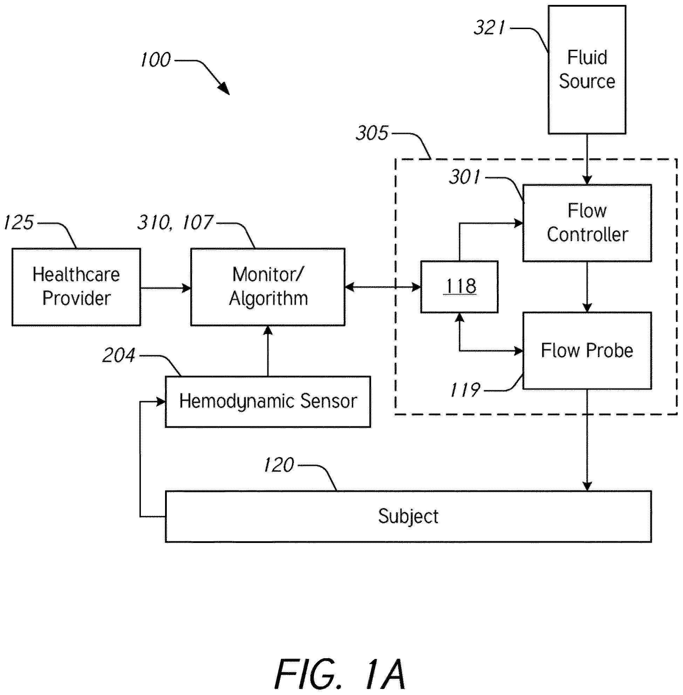

FIG. 1A illustrates a schematic flow diagram of a subject monitoring system 100. The subject monitoring system 100 includes flow probe 119 in fluid communication with a fluid source 321. The fluid source can be, for example, an IV-bag, another in-line source, or a combination of the two. The subject monitoring system 100 can, in some aspects, further include at least one sensor 204 coupled to a subject 120 and/or a flow controller 301, the sensor 204 and the flow controller 301 being electronically coupled to the system via a cable or via a wireless connection. In some embodiments, the at least one sensor 204 is a hemodynamic parameter sensor. In various embodiments, flow controller 301 and flow probe 119 are configured to couple with a processing algorithm 107 and/or monitor 310 via coupler/hub 118. Coupler/hub 118 provides electronic signaling in one or both directions between processing algorithm 107 and/or monitor 310 (and/or display device) and flow controller 301 and flow probe 119. The coupler/hub 118 can include at least a portion of the system electronics. The system electronics can be configured to power the flow probe 119 and/or the flow controller 301 and/or to detect, to transmit, to receive and/or to provide signal processing for the sensed data. In some embodiments, as shown by dotted area 305, the coupler/hub 118, flow controller 301, and flow probe 119 are configured for single use/disposable components of the system 100. The system 100 can be controlled or otherwise manipulated by a healthcare provider 125.

In various embodiments, as shown in FIG. 1A, the flow controller 301 is spatially separated, e.g., positioned "upstream" from the flow probe 119, where upstream refers to a relative position along a shared flow path relative to the subject 120. For example, the flow probe 119 can be positioned closer to the subject 120 relative to the flow controller 301 along the shared flow path from the fluid source 321. In this configuration, the flow controller 301 can selectively control the fluid source 321 (that can include one or more sources of infusion fluid) delivering fluid to the subject 120. Other sources of fluid infusion can be present in the system 100 provided that the other sources of fluid present in the system 100 are in combination with the fluid source 321 controlled by the flow controller 301 so that the fluid being delivered from the fluid source 321 and the other sources of fluid infusion pass through the flow probe 119.

In the system 100 with the upstream flow probe 119 and flow controller 301 arrangement, the healthcare provider 125 can be presented with a recommendation as to whether or not to accept the prompt generated by the algorithms 107, where the prompt can include, for example and without limitation, infuse fluid, alter an infusion rate, or terminate infusion to the subject 120. The clinician can accept the recommendation provided by the algorithms 107. Responsive to receiving an indication of acceptance, the system 100 can be configured to provide one or more signals to the flow controller 301 to adjust, to terminate or to otherwise manipulate the infusion rate of the one or more sources of infusion fluid to manage hemodynamic parameters of the subject 120 based on a combination of the flow probe data (e.g., provided by the flow probe 119) and/or subject hemodynamic parameter data (e.g., provided by the sensor 204).

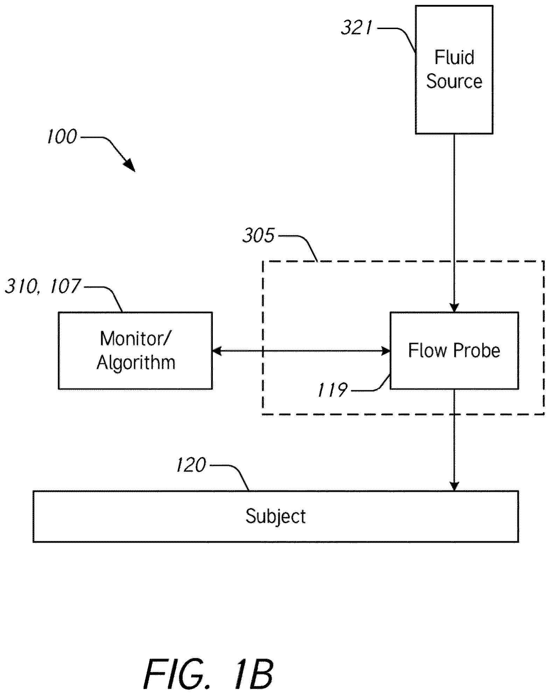

FIG. 1B illustrates another example embodiment of the subject monitoring system 100 with the system 100 including the flow probe 119 that senses fluid flow or mass flow of fluid delivery to the subject 120. Based at least in part on the sensed fluid or mass flow, the flow probe 119 provides flow-related data (e.g., fluid flow rate and/or mass flow rate) that the system 100 uses to derive a volume of fluid being delivered. The fluid can be delivered from the fluid source 321 that can include an IV bag, another in-line port, or a combination of the two. The monitor 310 and/or algorithm 107 can receive the flow-related data from the flow probe 119 and can derive fluid volume and/or fluid rate. This information can be displayed on the monitor 310 and/or the flow probe 119. A clinician can use this information to gain an understanding of the volume of fluid/mass the subject 120 has received. This system 100 finds applicability in determining an amount of fluid delivered, and is applicable without any flow controlling device or means.

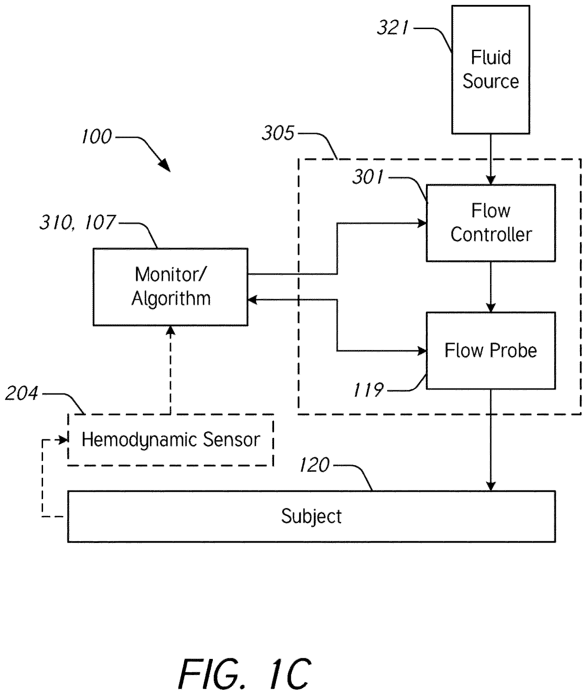

FIG. 1C illustrates another example embodiment of the subject monitoring system 100 with the system 100 including the flow probe 119 that senses fluid flow or mass flow of fluid delivery to the subject 120 and the flow controller 301 that controls the fluid flow from the fluid source 321 to the subject 120. The flow probe 119 provides flow-related data (e.g., fluid flow rate and/or mass flow rate) that the system 100 uses to derive a volume of fluid being delivered. The fluid can be delivered from the fluid source 321 that can include an IV bag, another in-line port, or a combination of the two. The monitor 310 and/or algorithm 107 can receive the flow-related data from the flow probe 119 and can derive fluid volume and/or fluid rate. This information can be displayed on the monitor 310 and/or the flow probe 119. A clinician can use this information to gain an understanding of the volume of fluid/mass the subject 120 has received. In addition, this information can be used in a feedback loop to control the fluid flow via the flow controller 301.

In some embodiments, the system 100 also includes the physiological sensor 204 that provides physiological data to the algorithm 107. The physiological sensor 204 can be a hemodynamic sensor, for example. The hemodynamic sensor can be the FLOTRAC^{®} sensor, in certain implementations. The physiological sensor 204 can be configured to provide information capable of being transformed into one or more forms of heart output data. In some embodiments, an oximetry device can be used as part of the physiological sensor 204. In certain embodiments, the oximetry device can be a finger cuff device that is integrated with the system 100, the system electronics, and/or the monitor 310 and/or the algorithm 107. The system 100 can utilize the physiological data in the algorithm 107 to determine how the subject 120 responds to administered fluid volumes. Based on the correlated sensed data from the flow probe 119 and the data from the physiological sensor 204, the algorithm 107 can determine the response of the subject 120, provide information (e.g., a recommendation) to the clinician regarding subsequent bolus administration, and/or control the amount and rate of volume of fluid delivered to the subject 120 using the flow controller 301.

In some embodiments, the flow controller 301 is operated independently of the sensed fluid flow (e.g., manually or electronically but without being part of a feedback loop using flow-related data). This may be useful to deliver a bolus to the subject at a desired time and/or for a desired duration. In some embodiments, although the flow controller 301 is configured to be controlled automatically by the algorithm 107, the system 100 can be configured to provide the clinician the ability to override the algorithm 107 and its administration protocol.

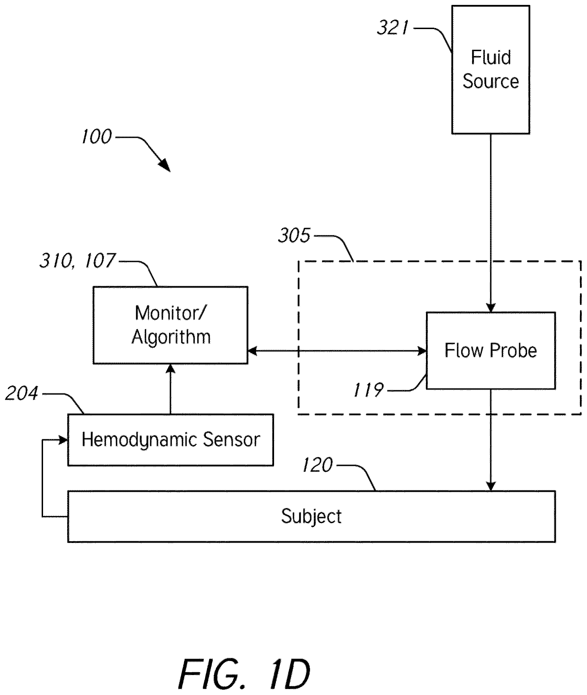

FIG. 1D illustrates an example embodiment of the subject monitoring system 100 that includes the flow probe 119, the physiological sensor 204, and the monitor 310 with the algorithm 107. The monitor 310 can, for example, include a graphical user interface (GUI). In the system 100, the flow probe 119 can be configured to sense flow-related information in combination with sensed physiological data from the physiological sensor 204. In such implementations, the physiological sensor information (e.g., provided by the physiological sensor 204) can be utilized along with the fluid delivery related data (e.g., provided by the flow probe 119) to generate a recommendation to a clinician of one or more subsequent administration protocols that can include adjustments or targeted flow rates and/or fluid volumes. In various implementations, there is no automated flow controller or other automated device to automatically control administration of the fluid. Thus, in this aspect, it is in the discretion of the clinician to control the subsequent administration of fluid based on the recommendation provided by the system 100 (e.g., displayed or communicated using the monitor 310).

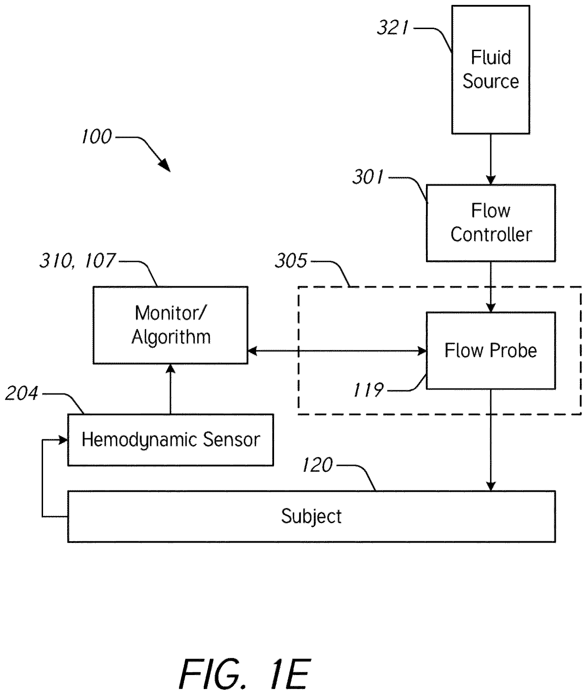

FIG. 1E illustrates another example embodiment of the subject monitoring system 100 that includes the flow probe 119, the physiological sensor 204 that provides physiological data to the algorithm 107 wherein the algorithm 107 utilizes the flow-related information and the physiological sensor data to provide recommendations regarding subsequent administration protocol(s) that can include a targeted flow rate and/or volume and/or adjustments to the flow rate and/or volume. The system 100 also includes the flow controller 301 in fluidic communication with the fluid source 321, that can be manually manipulated to vary fluid delivery rate and, by extension, the volume of fluid delivered. The flow controller 301 is configured to be controlled by the clinician manually. The actual physical control of fluid administered can be a standard IV roller clamp, for example. Such manual control of fluid delivery may be part of a standard IV tubing set that can be connected and/or adapted for use with the system 100. Thus, it may be in the discretion of the clinician to control the subsequent administration of fluid based on the recommendation provided by the system (e.g., through the algorithm 107 and the monitor 310).

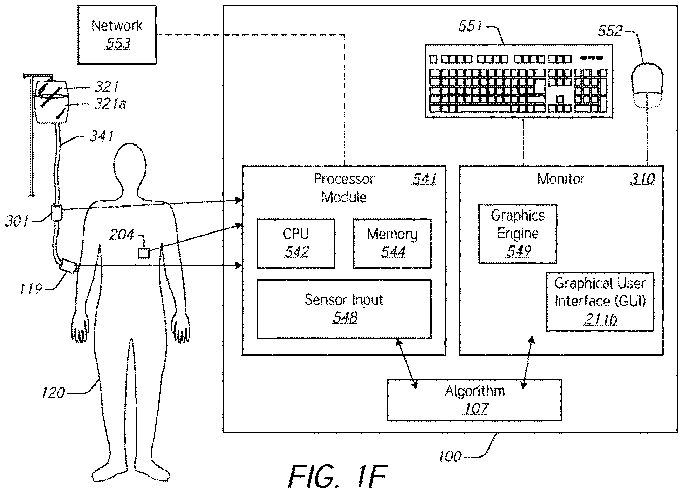

FIG. 1F illustrates a schematic diagram of the subject monitoring system 100 according to FIG. 1A. The subject monitoring system 100 includes at least one sensor 204 coupled to a subject 120. In some embodiments, the monitoring system 100 is a bed-side system, and can be integrated into an existing drug delivery stand, bedbox, or monitoring system rack. In certain implementations, the at least one sensor 204 is a hemodynamic parameter sensor. The system 100 also includes the flow probe 119. Sensor 204 and flow probe 119 are configured to couple with a processor module 541. In some embodiments, the processor module 541 is connected to a network 553, such as a wired or wireless network, to allow monitoring on a remote display (not shown). The memory unit 544 can be a volatile memory, such as flash memory, or non-volatile memory, such as read-only memory. In addition, the memory unit 544 can be a database that is located within the system 100, or alternatively, located remotely from the system 100. In some embodiments, the memory unit 544 can be located within or coupled to the monitor 310.

Processor module 541 is coupled to the monitor 310. Monitor 310 includes a graphics engine 549 and graphical user interface (GUI) 211b to render and display the signals received from the processor module 541. The graphics engine 549 and the GUI 211b outputs images and graphics corresponding to the physiological data to the monitor 310 or other display device. In some embodiments, the monitor 310 can be configured through the graphical user interface 211b to be touch-sensitive, and allows data or commands to be entered by an application of pressure, via, for example, a clinician's finger or a stylus, to the monitor 310. Furthermore, the monitor 310 can include a keyboard 551 for data input. The keyboard 551 can be a touch sensitive keyboard located on a portion of the monitor 310, or it can be an external hard keyboard coupled to the monitor 310. A mouse or pointing device 552 can be coupled to the monitor 310 and used to enter data or commands into the system 100. The monitor 310 can be configured to receive voice command instructions using dictation software stored on or in the processor module 541.

In some embodiments, the monitor 310 and the processor module 541 can be an integrated unit within a single housing. In certain embodiments, the processor module 541 can be separate from the monitor 310.

The processor module 541 and/or the monitor 310 can include one or more data storage devices comprising computer program code, e.g., algorithm 107, specifically configured such that when operated on by one or more electronic processors causes said one more electronic processors to perform operations, such as running code related to receiving and/or transmitting signals (e.g., to/from the sensors, telemetry components, display, user input devices, etc.), calculating values, interactions with graphical interfaces.

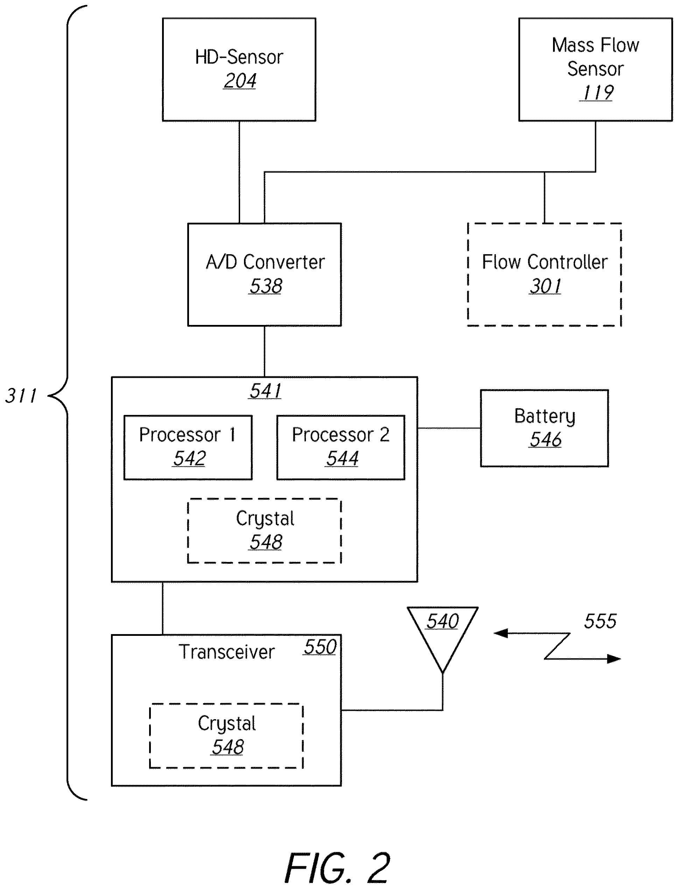

FIG. 2 illustrates system electronics 311 associated with the processor module 541 that acts as central control unit and that houses, for example, a first processor 542 and a second processor 544 (e.g., where the processor can be an EEPROM, SRAM). The processor module 541 is configured to control the processing of the system electronics 311. In certain embodiments, a computer system other than a microprocessor is used to process data as described herein. In some embodiments, the processors can include an application specific integrated circuit (ASIC) for some or all the central processing. The EEPROM 542 provides semi-permanent storage of data, for example, storing data such as sensor identifier (ID) and programming to process data streams. The SRAM 544 can be used for the system's cache memory, for example for temporarily storing recent sensor data. In some alternative embodiments, memory storage components comparable to EEPROM and SRAM may be used instead of or in addition to the disclosed hardware, such as dynamic RAM, non-static RAM, rewritable ROMs, flash memory.

A transceiver 550 (e.g., an RF transceiver) can be operably connected to the microprocessors 542, 544 to transmit the sensor data from either the HD-sensor 204 (e.g., hemodynamic sensor or physiological sensor) or the flow probe 119 to a receiver within a wireless transmission 555 via antenna 540. Although an RF transceiver is shown here, embodiments can include a wired rather than wireless connection to the receiver. A second quartz crystal 548 can provide the system time for synchronizing the data transmissions from the RF transceiver 550. It is noted that the transceiver 550 can be substituted with a transmitter in some embodiments. In some embodiments, other mechanisms such as optical, infrared radiation (IR), ultrasonic, may be used to transmit and/or receive data

In addition to receiving signals from the flow probe 119 and/or controlling fluid infusion, the processor module 541 includes systems and methods for receiving and processing signals such as an analog-to-digital (A/D) converter 538 to obtain one or more sensor values from either sensor independently or each of the hemodynamic parameter sensor 204 and the flow probe 119. In addition, the processor module 541 includes systems and methods for displaying the one or more sensor values from either or both the hemodynamic parameter sensor 204 and the flow probe 119.

With reference to FIG. 1F, the data or results can be displayed on the monitor 310, e.g., via a graphics engine 549. The processor module 541 can include systems and methods for sending and receiving one or more sensor signals or calculated values from the hemodynamic parameters sensor 204, the flow probe 119, and/or flow controller 301 to a network 553 via the Internet, intranet, or telecommunication system.

The telemetry (e.g., radio telemetry) devices contemplated for use in conjunction with either of the hemodynamic parameter sensing device, flow probe, and flow controller possess features including small package size, adequate battery life, acceptable noise-free transmission range, freedom from electrical interference, and easy data collection and processing. Telemetry provides several advantages, including the ability of an implanted or inserted device to measure hemodynamic parameter values in a sealed-off, sterile environment. The present disclosure is not limited by the nature of the telemetry equipment or methods for its use. Indeed, commercially available equipment can be modified for use with the devices of the present disclosure. Similarly, custom-designed telemetry devices used in hemodynamic parameter monitoring can be used in conjunction with the hemodynamic parameter sensing devices of the present disclosure. In some implementations, transmitters can be configured (including programming) with an external magnet to transmit at 4-, 32-, or 256-second intervals, with battery lifetimes at the current longest transmission intervals (about 256 seconds) approximately up to two years or more. In various implementations, transmitters, along with the hemodynamic parameter sensing device, are configured as "disposables," with lifetimes of days, weeks, or months.

With reference to FIG. 2, a battery 546 or other power source is operably connected to the microprocessor 542 and provides power for sensor 204, flow probe 119, and/or flow controller 301. In some embodiments, the battery 546 is rechargeable. In various embodiments, a plurality of batteries can be used to power the system. In certain embodiments, one or more capacitors can be used to power the system. A quartz crystal 548 may be operably connected to the processor module 541 to maintain system time for the system as a whole.

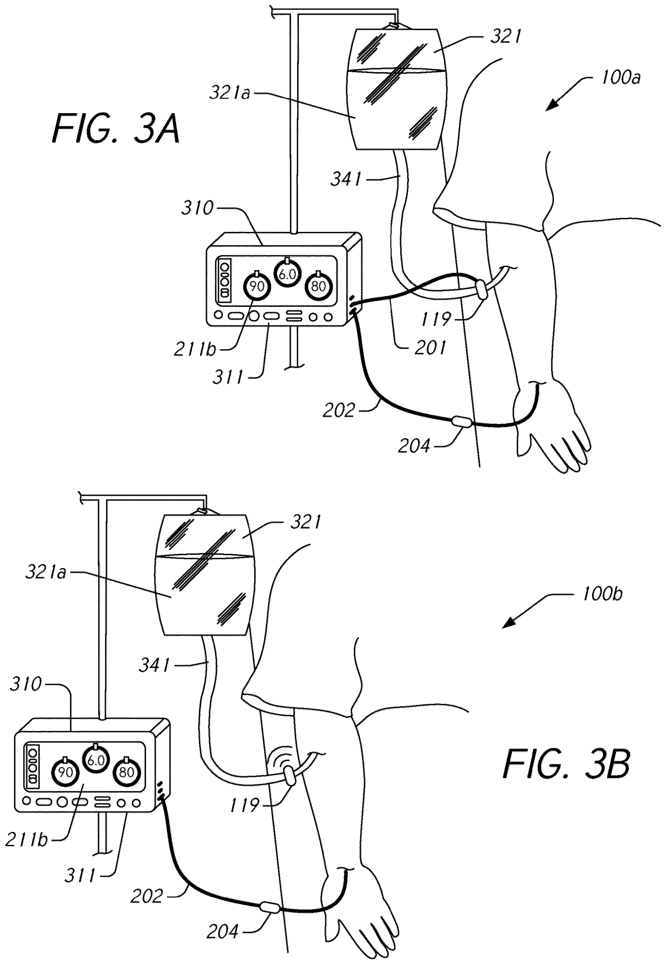

FIGS. 3A and 3B illustrate example embodiments of subject monitoring systems described herein. FIG. 3A depicts an integrated system 100a (as a variation of system 100) for hemodynamic management. FIG. 3B depicts a partially wireless system 100b (as a variation of system 100) for hemodynamic management. Systems 100a, 100b comprises a hemodynamic parameter data sensor 204 (also referred to as "HD-sensor" or "hemodynamic parameter sensor") and a flow probe 119 (also referred to as "mass flow sensor" or "flow rate sensor") with fluid 321a being infused via an IV bag 321, for example, into a subject via tubing 341. Referring to FIGS. 3A and 3B, the systems 100a, 100b include a flow probe 119 that is wired or wirelessly coupled to the monitor 310, respectively.

FIG. 3A illustrates the flow probe 119 with a dedicated system cable 201 to the electronic module, communication module, and/or monitor 310. While system 100a can include an HD-sensor and a flow-sensor of any type, the discussion hereinafter is directed to pressure sensors and magnetic or ultrasonic flow sensors, as an example embodiment of the system 100a. Thus, the system 100a includes HD-sensor 204, a vascular access device, such as a catheter, and the flow probe 119 along the flow path of the catheter and coupled to system electronics 311 or otherwise coupled to a processor module and/or the monitor 310. In some embodiments, the HD-sensor 204 is a pressure sensor configured and arranged to generate a signal associated with or corresponding to the blood pressure of the circulatory system of a subject.

The vascular access device (not shown) such as a catheter, is connected to an infusion source 321, such as an IV bag, containing an infusion fluid 321a, via tubing 341. The infusion source 321 is capable of infusing fluid into the subject via gravity, or at a rate of about 2 L/hour to about 6 L/hour. The tubing 341 is fluidically coupled with the flow probe 119, which is configured to determine the mass/time rate or total volume of one or more infused fluids into the subject and is responsive to the system electronics 311. The vascular access device coupled to the tubing 341 may comprise multiple ports or lumens for the introduction of one or more fluids independent of or in cooperation with the contents of the gravity feed IV bag. For example, the vascular access device can include a Swan-Ganz type catheter implanted in the subject's circulatory system (e.g., a vein or artery), and/or the flow probe 119 connected to the catheter can be configured to detect and/or monitor total fluid introduction to the subject from one of the access ports associated with the Swan-Ganz catheter.

With reference to FIGS. 3A and 3B, the systems 100a, 100b include system electronics 311 operably connected to one or both of the HD-sensor 204 and the flow probe 119, and configured to receive and/or process one or more signals generated by the HD-sensor 204 and/or the flow probe 119. The one or more signals can be associated with a hemodynamic parameter (e.g., a static or a dynamic value) of the subject's hemodynamic state and the mass flow rate or total mass volume infused into the subject.

In FIG. 3A, the system electronics 311 are operably coupled to electronic cable 201, which in turn can be operably coupled to the flow probe 119, such as but not limited to an ultrasonic or magnetic flow probe or device that measures the weight (load) of an IV bag and provides continuous, intermittent, or on-demand estimates to the system electronics 311 and/or algorithm of the weight of the bag for determining infusion volume and/or infusion rate.

With continued reference to FIG. 3A, an electronic cable 201 is operably coupled to the system electronics 311 and monitor 310 and is configured and arranged to operably connect with the flow probe 119. Alternatively, as in system 100b depicted in FIG. 3B, wireless communication operably couples the monitor 310 and flow probe 119. The system can include a unique and/or interactive display/monitor 211b, which can be configured to communicate with the systems 100a, 100b by wired and/or wireless transceiver components known in the art.

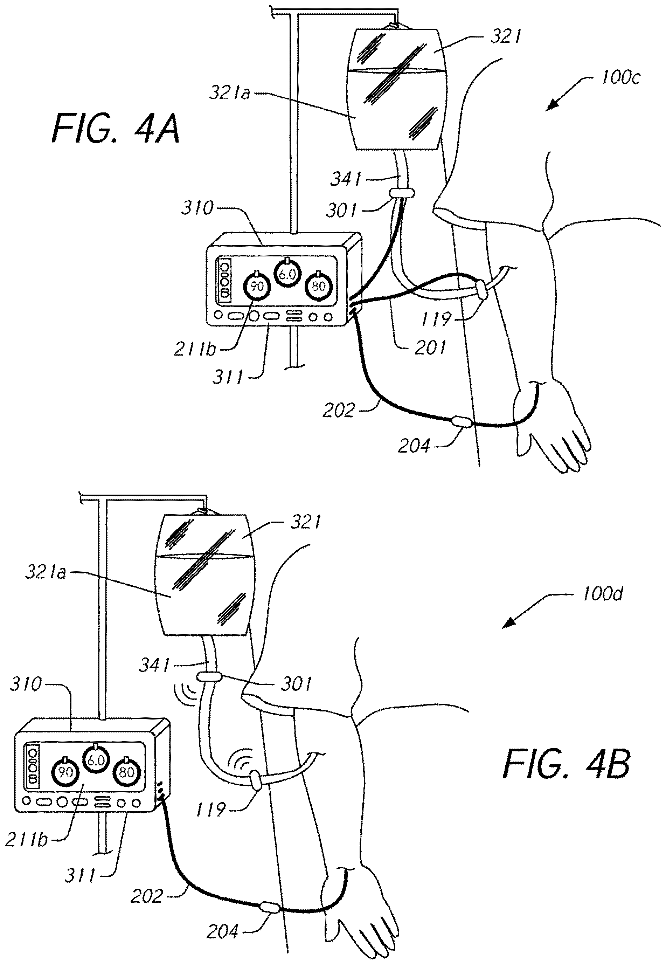

FIGS. 4A and 4B illustrate example infusion systems 100c, 100d, respectively, that include an apparatus configured to control delivery of fluid. The apparatus is coupled to a processor module, an algorithm, and/or the monitor 310. Systems 100c, 100d are similar to the systems 100a, 100b with the addition of the flow controller 301.

A process controller of the systems 100c, 100d determines a first effect on a physiological parameter of the subject associated with administration to the subject of a first fluid bolus corresponding to a first mass flow condition, and stores first administration-related data relating to the first effect within device storage. The first mass flow condition can include infusion of a fluid to the subject at a rate of about 2 L/hour to about 6 L/hour. The process controller then determines a second effect on the physiological parameter of the subject associated with administration to the subject of a second fluid bolus corresponding to a second mass flow condition, and stores second administration-related data relating to the second effect within the device storage. The second mass flow condition can include infusion of a fluid to the subject at a rate the same or different from the first mass flow condition by sending a signal to the flow controller 301 to adjust the flow rate of fluid 321a. The process controller provides a fluid administration signal based upon at least one of the first administration-related data or the second administration-related data. In some embodiments, the fluid administration signal is directed to the flow controller 301.

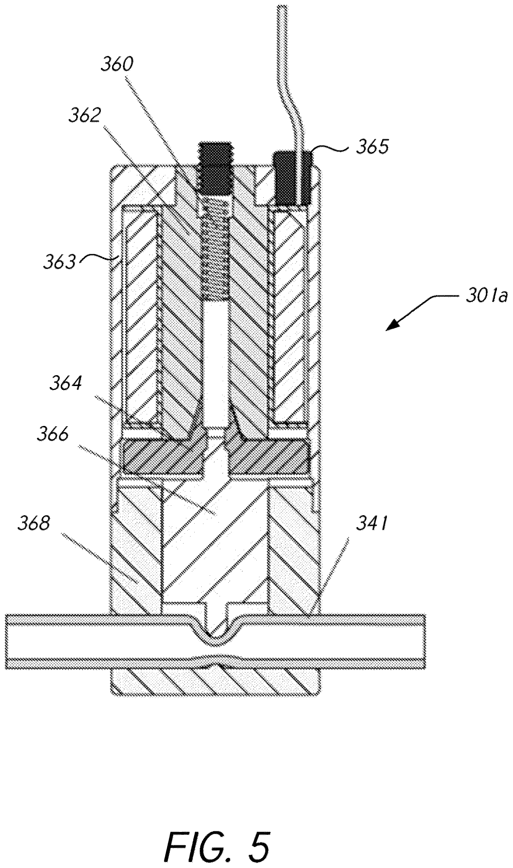

FIG. 5 depicts an example manual flow controller 301a that includes a solenoid-based, "pinch valve." Flow controller 301a includes a solenoid 362 electrically coupled to energizing means 365, solenoid 362 encased in housing 363 coupled to body 368, solenoid 362 having biasing spring 360 coupled to armature 364 for driving plunger 366 against tubing 341 to compress the tubing and restrict fluid flow therethrough. Energizing solenoid 362 via energizing means 365 causes retraction of the plunger 366 for allowing unrestricted fluid flow through tubing 341 receiving fluid from IV bag 321. The energizing solenoid 362 causes spring to bias plunger and reduce the internal diameter of tubing 341 to restrict fluid flow. In some embodiments, the flow controller 301a can be configured such that unrestricted fluid flow is provided in the de-energized state (reversed configuration as previously described). Energizing means 365 can be configured for control by an algorithm (e.g., the algorithm 107 described herein) and can include user override and/or other safety protocols. Other types of pinch valve flow controllers can be used. Pinch valve flow controllers are commercially available, for example, from BIO-Chem, Neptune Research, ASCO Valve Inc., Clippard Instrument Laboratory, and Valcor Engineering Corp.

With reference to FIGS. 4A and 4B, the systems 100c, 100d can be configured to incorporate one or more algorithms that receive and/or transmit information from the hemodynamic parameter sensor 204, flow probe 119, and flow controller 301 for providing methods of improved patient outcomes, assisted fluid delivery, and/or automated fluid optimization while using a clinician's preferred workflow. The systems 100c, 100d can be used with methods for open-loop and/or closed-loop patient-adaptive hemodynamic management, such as those disclosed in U.S. Patent No. 8,617,135, entitled "System and Method for Closed-Loop Patient-Adaptive Hemodynamic Management," issued December 31, 2013.

In some embodiments, the HD-sensor 204 is a pressure sensor that generates a signal associated with or corresponding to the blood pressure of the circulatory system of a subject and the flow probe 119 is a magnetic or ultrasonic mass flow measuring device that generates a signal associated with the infusion rate and/or infusion volume of one or more fluids introduced to a patient through the catheter or tubing. Other means of monitoring the flow rate or mass of fluid can be used, such as a mass or weight method.

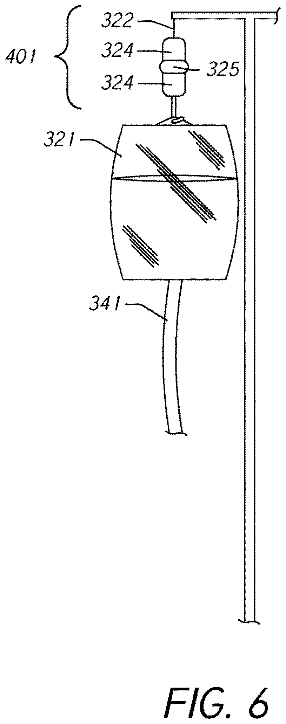

For example, FIG. 6 depicts and IV bag weight monitoring device 401 shown attached to a stand and an IV bag. IV bag weight monitoring device 401 includes a load cell 325 with vertically opposing projecting members 324, one member connected to a coupler 322 for suspending from a stand, and the other member for connecting to the IV bag. Load cell 325 are available commercially such as an iLoad Mini Sensor (LoadStar Sensors (Fremont, CA)) or a LSB200 Miniature-Beam Load Scale (FUTEK Advanced Sensor Technology, Inc., Irvine CA). The IV bag weight monitoring device 401 is configured to provide IV fluid levels/flow rates based on the weight of the IV bag and its contents. The IV bag weight monitoring device 401 monitors the weight of the IV bag and provides a signal to an interface that can calculate the amount of fluid present and the rate of dispensing of the fluid per minute. The IV bag weight monitoring device 401 interface can be configured to provide signals to a processor module (e.g., the processor module 541 described herein) for use by an algorithm (e.g., the algorithms 107 described herein) and/or as a user-friendly GUI interface to display on a monitor (e.g., the monitor 310 described herein). In some embodiments, the IV bag weight monitoring device 401 provides a signal to a processor module, algorithm, and/or monitor as an alternative to signals otherwise provided by a flow probe (e.g. the flow probe 119 described herein). In some embodiments, the IV bag weight monitoring device 401 provides a signal to the processor module, algorithm, and/or monitor in addition to signals provided by the flow probe.

Thus, the systems described herein provide for a clinical device that is capable of administering fluids, determining infusion volumes and/or rates, controlling infusion, and providing for the administration of fluids, (e.g., blood products, and medications), such administrations being calculated and/or controlled by an algorithm, monitoring the subject response to fluids administered, and displaying one or more of the sensed parameters, mass flow rates of infusion fluids, calculated values, and dynamically, intermittently, or continuously monitored information to the healthcare provider. The disclosed systems provide for the automation and standardization of administration of intravenous fluids, blood products, and/or blood-pressure modifying medications, for example, to improve patient outcomes.

The disclosed apparatuses, methods, and systems also provide devices and systems configured to administer or direct the administration of anesthetics or for use in anesthesiology, among other healthcare related functions.

The systems, in combination with the disclosed methods, is capable of improving methods of dynamically adapting to specific subjects using known biases in conjunction with associated physiological parameters, their means and standard deviations in relationship to one another, and observed responses to previous interventions by the systems; determining whether a fluid bolus (of an amount and/or at a specified infusion rate) and/or blood-pressure adjusting medications are indicated; and, if so, administering them or providing an indication to the healthcare provider to administer them. The systems are also configured for monitoring responses in combination with mass flow data and adapting to the subject, and determining whether additional fluid bolus and/or blood product administration is indicated, and, if so, administering them or providing an indication to the healthcare provider to administer them.

The disclosed apparatuses, methods, and systems also provide for improved enhanced learning and adaptation by data (of a multitude of subjects) shared between devices over time to improve the algorithm of the systems and to improve expectations across patient populations. The use of fluid administration volumes, for example, at rates of about 2 L/hour to about 6 L/hour in the adapting process provides for improved dynamic and/or real-time adjustments to future fluid administration volumes and/or rates and their thresholds, as well as improved control of the administration dose and threshold of medication, as well as automatic adjustments to the weight of each measured parameter in decision-making by the algorithm of the apparatus.

Using a combination of the change in fluid predictive parameters and the change in cardiac output in response to a bolus delivered at rates of about 2 L/hour to about 6 L/hour corresponding to a specific mass flow rate or volume allows an improved measurement of the bias present in a particular subject at a particular time and allows for subject-adaptive responses to be addressed more accurately. Using a combination of the change in the fluid predictive parameters and the change in cardiac output in response to a bolus corresponding to a mass flow rate or volume infused at about 2 L/hour to about 6 L/hour provides for a dynamically accurate measurement of the bias present in a particular subject at a particular time and allows for subject-adaptive responses to be addressed and/or improved.

The disclosed apparatuses, systems and methods provide for one or more hemodynamic parameters to be used in combination with high infusion volumes and/or high infusion rates, the hemodynamic parameters corresponding to cardiac output information, for administration of fluids and/or pharmacologic agents, such as blood-pressure affecting drugs, among other things. Thus, the disclosed systems and methods include a device capable of administering or instructing the administration of IV fluids, blood, and/or medications to subjects autonomously and a set of processes for measuring and/or controlling fluid administration volume to achieve a target or predicted hemodynamic profile. The method includes receiving administration-related data relating to one or more physiological processes of a subject corresponding to infusion rates of about 2 L/hour to about 6 L/hour. The method also includes determining, based at least in part upon the administration-related data, administration-related data associated with a current state of the subject. The method also includes adjusting, using a processor and algorithm, administration of fluid to the subject based at least in part upon the administration-related data.

The systems and devices include one or more processors and memory operatively coupled to the one or more processors. The memory stores signals from at least one hemodynamic sensor and at least one flow probe which, when executed by the one or more processors, cause the one or more processors to receive administration-related data relating to one or more physiological processes of the subject and mass flow data, and to determine, based at least in part upon the administration-related data, administration-related data associated with a current state of the subject. The signals further cause the one or more processors to adjust (directly or indirectly) administration of fluid to the subject based at least in part upon the administration-related data.

### Example Methods for the Administration of Fluid to a Subject

The disclosed systems can be configured to carry out one or more computer-implemented methods. In a first aspect, a computer-implemented method for facilitating the administration of fluid to a subject is provided. The computer-implemented method includes determining a first effect on a physiological parameter of the subject associated with administration to the subject of a first fluid bolus event under a first mass flow condition, and then storing, using a processor, first administration-related data relating to the first effect corresponding to the first mass flow condition. In some embodiments, the first mass flow condition includes infusion of a fluid to the subject at a rate of about 2 L/hour to about 6 L/hour.

The method further includes determining a second effect on the physiological parameter of the subject associated with administration to the subject of a second fluid bolus event corresponding to a second mass flow condition, and then storing, using a processor, second administration-related data relating to the second effect corresponding to the second mass flow condition. In some embodiments, the second mass flow condition comprises infusion of a fluid to the subject at a rate of about 2 L/hour to about 6 L/hour. The method further includes generating, using the processor, a fluid administration signal based at least in part upon the first administration-related data and the second administration-related data on the physiological parameter of the subjects. In some implementations, the fluid administration signal is directed to a flow controller.

In some embodiments, a method is provided comprising a device that includes one or more processors, and a memory operatively coupled to the one or more processors, the memory storing program code which, when executed by the one or more processors, determines a first effect on a physiological parameter of the subject associated with the method of administration to the subject of a first fluid bolus corresponding to a first mass flow condition, and storing first administration-related data relating to the first effect within device storage. In some embodiments, the first mass flow condition comprises infusion of a fluid to the subject at a rate of about 2 L/hour to about 6 L/hour. The method further comprises providing for the program code determining a second effect on the physiological parameter of the subject associated with administration to the subject of a second fluid bolus corresponding to a second mass flow condition, storing second administration-related data relating to the second effect within the device storage, and generating a fluid administration signal based upon at least one of the first administration-related data and the second administration-related data. In some embodiments, the second mass flow condition comprises infusion of a fluid to the subject at a rate of about 2 L/hour to about 6 L/hour. In some embodiments, the fluid administration signal is directed to a flow controller. In some embodiments, the first and the second bolus related data is obtained from an infusion to the subject at a rate of about 6 liters/hour.

In certain embodiments, a computer-implemented method for providing clinical decision support relating to the administration of fluid to a subject at a rate of about 2 L/hour to about 6 L/hour is provided. The method includes receiving administration-related data comprising sensor data corresponding to one or more physiological processes of a subject corresponding to at least one infusion mass flow condition, determining, based at least in part upon the administration-related data, administration-related data associated with a current state of the subject, and providing, using a processor, a fluid administration recommendation based at least in part upon the administration-related data.

In various embodiments, a computer-implemented method for providing clinical decision support relating to the administration of fluid to a subject at a rate of about 2 L/hour to about 6 L/hour is provided. The method comprises determining a first effect on a physiological parameter of the subject associated with an administration of a first fluid bolus to the subject corresponding to a first mass flow condition, storing, using a processor, first administration related data relating to the first effect, determining a second effect on the physiological parameter of the subject associated with administration of a second fluid bolus to the subject corresponding to a second mass flow condition, storing, using a processor, second administration-related data relating to the second effect, and providing, using the processor, a fluid administration recommendation based at least in part upon at least one of the first administration-related data and the second administration-related data.

In some embodiments, a computer-implemented method for providing clinical decision support relating to the administration of fluid to a subject is provided. The method includes receiving administration-related data relating to one or more effects on a state of the subject associated with prior administration of fluid to the subject corresponding to a prior mass flow condition of infusion of a fluid to the subject at a rate of about 2 L/hour to about 6 L/hour, then determining, using a processor and based upon the administration-related data, a predicted change in a physiological parameter of the subject in response to the administration of a fluid bolus to the subject. The method further includes providing, using the processor, a fluid administration recommendation corresponding to a mass flow condition based upon the predicted change.

In some embodiments, a computer-implemented method for facilitating the administration of fluid bolus to a subject is provided. The method includes receiving administration-related data relating to one or more effects on a state of the subject associated with prior administration of a fluid bolus to the subject corresponding to a prior mass flow condition of infusion of a fluid to the subject at a rate of about 2 L/hour to about 6 L/hour, and determining, using a processor, and based upon the administration-related data, a predicted change in a physiological parameter of the subject in response to the administration of a fluid bolus to the subject corresponding to a new mass flow condition of infusion of a fluid to the subject at a rate of about 2 L/hour to about 6 L/hour. The method further includes generating, using the processor, a fluid administration signal corresponding to the new mass flow condition based upon the predicted change. In some embodiments, the fluid administration signal is directed to a flow controller.

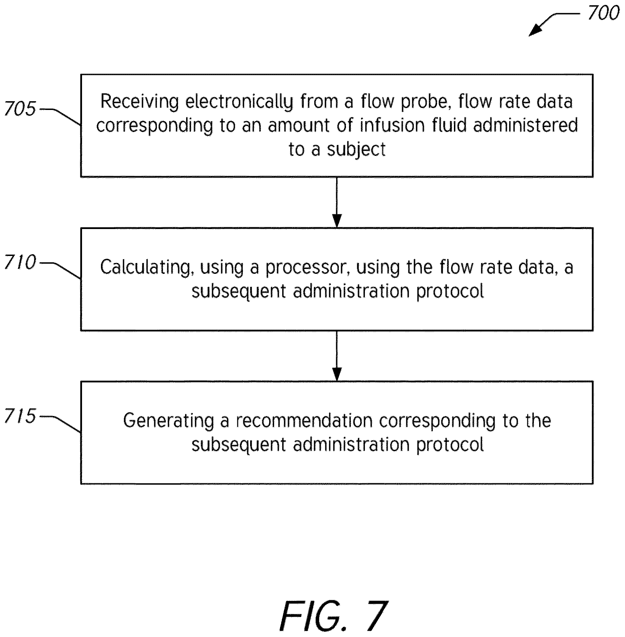

FIG. 7 illustrates a flowchart of an example process 700 for providing assisted delivery management in combination with the systems, apparatuses, and methods disclosed herein. The process 700 can be performed by any of the subject monitoring systems 100 illustrated in FIGS. 1A-1F and FIG. 2 and/or the systems 100a, 100b, 100c, 100d of FIGS. 3A, 3B, 4A, and 4B. Accordingly, for ease of description, the process 700 is described as being performed by the system and/or a processor of the system. However, it is to be understood that the process 700 can be performed by any component or combination of components of the systems described herein. Similarly, any step, portion of a step, or combination of steps in the process 700 can be performed by any component or combination of components of the systems described herein. The process 700 is an example and not intended to limit the scope of the disclosure. The process 700 can be altered, e.g., by having steps added, removed, rearranged, combined and/or performed concurrently.

The process 700 begins at step 705 where the system receives electronically from a flow probe, flow rate data corresponding to an amount of infusion fluid administered to a subject. In some embodiments, the amount of the infusion fluid administered is configured to alter or maintain a physiological state of the subject. At step 710, the processor calculates, using the flow rate data, a subsequent administration protocol. At step 715, the processor generates a recommendation corresponding to the subsequent administration protocol. In some embodiments, the recommendation comprises a visual, audio, or textual presentation of the amount and/or duration of administration for a health care provider to manually act on or ignore.

In some embodiments, the representation is provided on a user interface which corresponds to the fluid administration protocol for the health care provider. The user interface provides a visual, audio, or textual adaptation of the subsequent administration protocol for which the healthcare provider has full discretion as to comply or not.

In some embodiments, the administration protocol corresponds to providing an infusion of a fluid to the subject at a rate corresponding to a gravity-assisted delivery system, such as an IV bag. The administration protocol can be an electronic signal to a monitor or display (a user interface) to provide graphical information or digitally created language that functions as a recommendation to the clinician as to subsequent fluid administrations, or the electronic signal can be presented to a flow controller to stop a fluid bolus currently being administered, or to continue a current action and continue to acquire physiological data and/or mass flow (rate) parameters, or to start a new fluid bolus, modify and/or or continue an existing fluid bolus or medication administration if one is being administered and/or to provide a maximum fluid bolus. The received physiological data can be used by a processor with an algorithm to provide parameters ("physiological parameters") such as hemodynamic data, e.g., cardiac output, stroke volume, heart rate, blood pressure and arterial pressure, for example.

In some implementations, the user interface provides a recommendation regarding a subsequent administration protocol. In some implementations, the user interface provides a recommendation regarding the subsequent administration protocol and is configured to receive a user input, e.g., indicating acceptance or rejection of the intervention command and for which the system provides an electronic signal to the flow controller. For example, a selection of fluid volume rates can be presented for the user to select/accept, or for the user to input a specific value using a keyboard. Additional graphical displays or representations can be employed and may comprise a standard "Starling Curve" of ventricular function, a graphical indicator of where the subject is perceived to be presently along that curve, and a band showing the ideal range of the curve for the subject, among other representations. In addition, minimum and maximum curves can be displayed showing the observed ranges of cardiac function in a given subject (not shown).

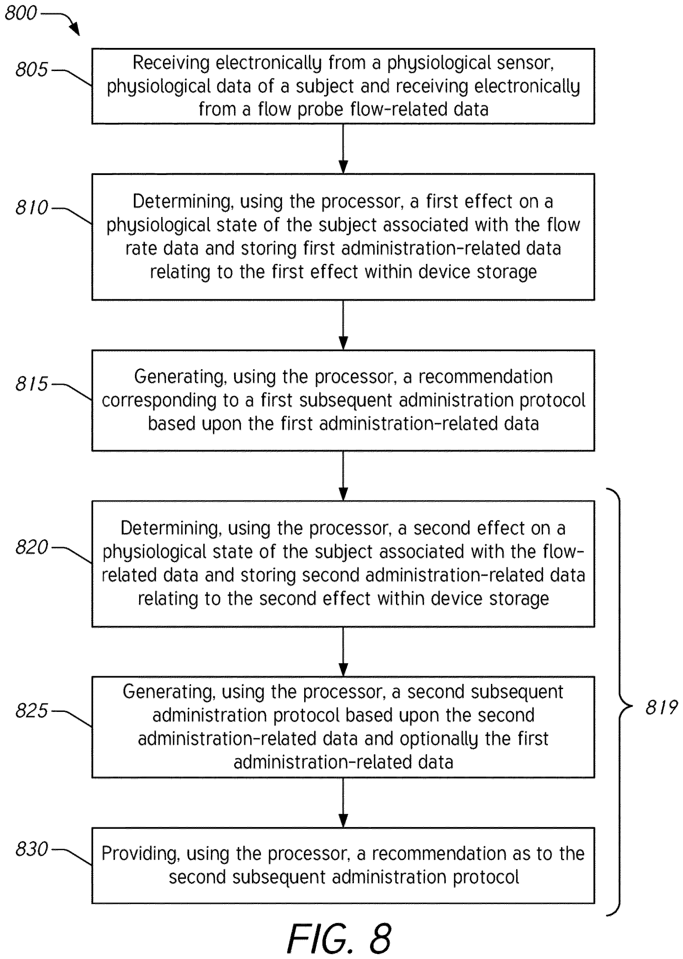

FIG. 8 illustrates a flowchart of an example process 800 for providing assisted delivery management in combination with the systems, apparatuses, and methods disclosed herein. The process 800 can be performed by any of the subject monitoring systems 100 illustrated in FIGS. 1A-1F and FIG. 2 and/or the systems 100a, 100b, 100c, 100d of FIGS. 3A, 3B, 4A, and 4B. Accordingly, for ease of description, the process 800 is described as being performed by the system and/or a processor of the system. However, it is to be understood that the process 800 can be performed by any component or combination of components of the systems described herein. Similarly, any step, portion of a step, or combination of steps in the process 800 can be performed by any component or combination of components of the systems described herein. The process 800 is an example and not intended to limit the scope of the disclosure. The process 800 can be altered, e.g., by having steps added, removed, rearranged, combined and/or performed concurrently.

The process 800 begins at step 805 where the system receives electronically from a physiological sensor, physiological data and receives electronically from a flow probe, flow related data. The flow related data corresponds to an amount of fluid administered to a subject. In some embodiments, the amount of the fluid administered is configured to alter or maintain a physiological state of the subject. At step 810, the processor determines a first effect on a physiological state of the subject associated with the flow related data and stores first administration related data relating to the first effect within device storage. At step 815, the processor generates a recommendation corresponding to a subsequent administration protocol based upon the first administration related data. In some embodiments, the recommendation comprises a visual, audio, or textual presentation of the amount and/or duration of administration for a health care provider to manually act on or ignore.

Optional steps 819 (encompassing steps 820, 825, and 830) includes step 820 where the processor determines a second effect on a physiological state of the subject associated with the flow related data and stores second administration related data relating to the second effect within device storage. At step 825, the processor generates a second subsequent administration protocol based upon the second administration related data and optionally the first administration related data. At step 830, the processor provides a recommendation as to the second subsequent administration protocol in a manner similar to that described above.

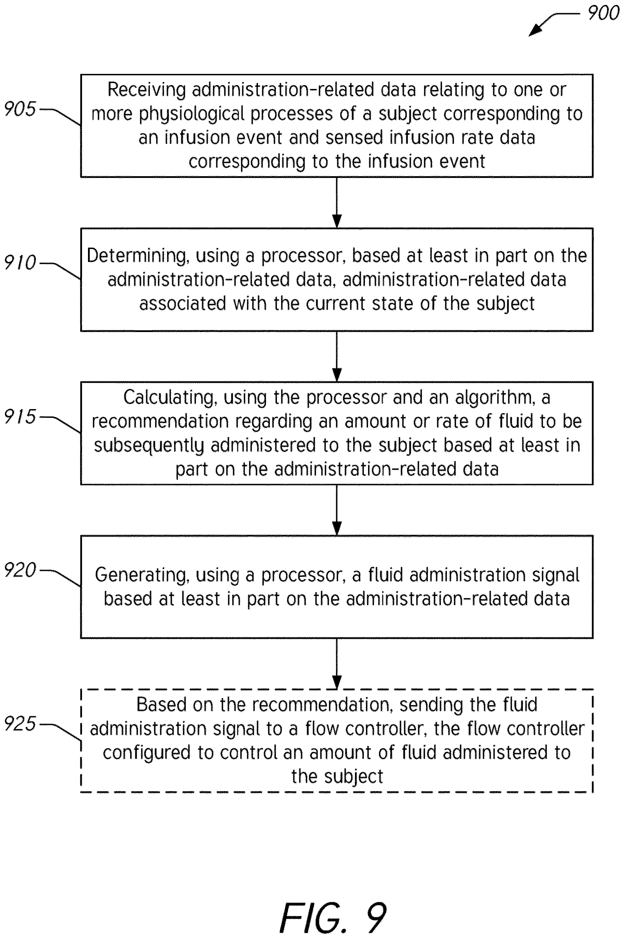

FIG. 9 illustrates a flowchart of another example process 900 for providing assisted fluid delivery management in combination with the systems, apparatuses, and methods disclosed herein. The process 900 can be performed by any of the subject monitoring systems 100 illustrated in FIGS. 1A-1F and FIG. 2 and/or the systems 100a, 100b, 100c, 100d of FIGS. 3A, 3B, 4A, and 4B. Accordingly, for ease of description, the process 900 is described as being performed by the system and/or a processor of the system. However, it is to be understood that the process 900 can be performed by any component or combination of components of the systems described herein. Similarly, any step, portion of a step, or combination of steps in the process 900 can be performed by any component or combination of components of the systems described herein. The process 900 is an example and not intended to limit the scope of the disclosure. The process 900 can be altered, e.g., by having steps added, removed, rearranged, combined and/or performed concurrently.

The process 900 starts at step 905 where the system (e.g., a processing module) using sensor input data receives administration-related data relating to one or more physiological processes of a subject corresponding to an infusion event and sensed infusion rate data corresponding to the infusion event. At step 910, the system can then determine, using a processor, and based at least in part upon the administration-related data, administration-related data associated with the current state of the subject. For example, the current state can be a hemodynamic state. At step 915, the processor in combination with an algorithm, can calculate an amount of fluid to be administered to the subject based at least in part upon the administration-related data. At step 920, the processor can generate a fluid administration signal based at least in part upon the first administration-related data. At step 925, the administration signal can optionally be sent to a flow controller, the flow controller configured to control an amount of infusion fluid to be administered to the subject.

The process 900 can be configured such that the user interface receives user input indicating acceptance or rejection of the fluid administration signal, where upon acceptance of the fluid administration signal a control signal for the flow controller is generated.

In some embodiments, the processor can be configured to calculate a predicted change in the subject's physiological parameter. A predicted change in the physiological parameter in response to administration of a first or second bolus to the subject can be determined using a process based at least in part on one or more of a subject-population database or a patient history record.

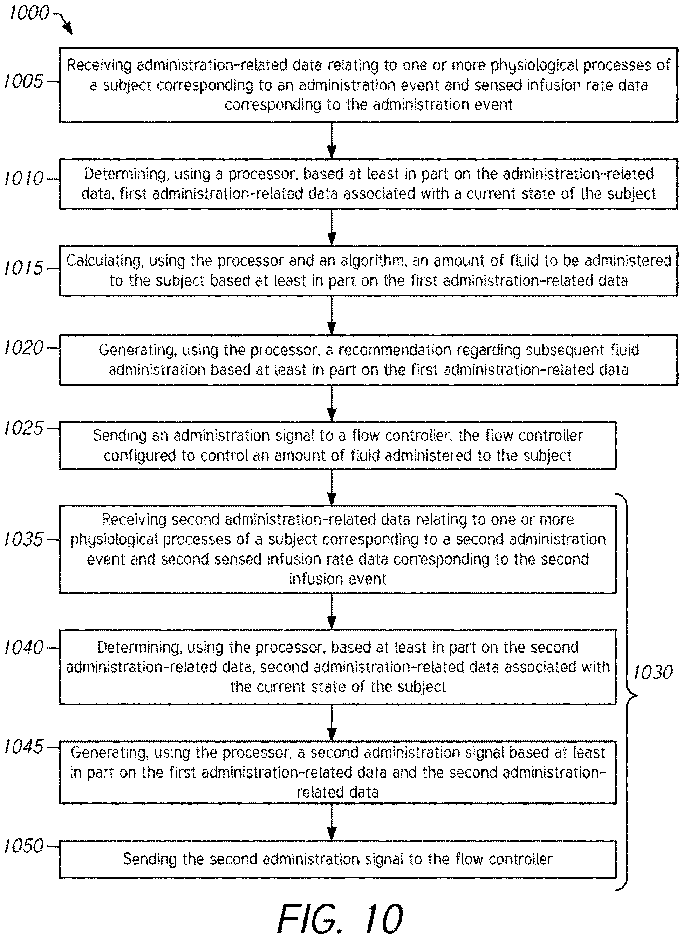

FIG. 10 illustrates a flowchart of an example process 1000 for providing assisted fluid delivery management in accordance with the systems, apparatuses, and methods disclosed herein. The process 1000 can be performed by any of the subject monitoring systems 100 illustrated in FIGS. 1A-1F and FIG. 2 and/or the systems 100a, 100b, 100c, 100d of FIGS. 3A, 3B, 4A, and 4B. Accordingly, for ease of description, the process 1000 is described as being performed by the system and/or a processor of the system. However, it is to be understood that the process 1000 can be performed by any component or combination of components of the systems described herein. Similarly, any step, portion of a step, or combination of steps in the process 1000 can be performed by any component or combination of components of the systems described herein. The process 1000 is an example and not intended to limit the scope of the disclosure. The process 1000 can be altered, e.g., by having steps added, removed, rearranged, combined and/or performed concurrently.

The process 1000 begins at step 1005 where the system (e.g., a processing module) using sensor input data receives administration-related data relating to one or more physiological processes of a subject corresponding to an administration event and sensed infusion rate data corresponding to the administration event. At step 1010, the system can then determine, using a processor, and based at least in part upon the administration-related data, first administration-related data associated with the current state of the subject. For example, the current state can be a hemodynamic state. At step 1015, the processor in combination with an algorithm, calculates an amount of fluid to be administered to the subject based at least in part upon the first administration-related data. At step 1020, the processor generates a recommendation regarding subsequent fluid administration based at least in part on the first administration-related data. At step 1025, an administration signal can be sent to a flow controller, the flow controller configured to control an amount of infusion fluid to be administered to the subject.

In some embodiments, process 1000 can further provide additional processing steps 1030. At step 1035, second administration-related data can be received relating to one or more physiological processes of a subject corresponding to a second administration event and second sensed infusion rate corresponding to the second administration event. At step 1040, the processor can determine, based at least in part upon the second administration-related data, second administration-related data associated with the current state of the subject. At step 1045, the processor can generate a second administration signal based at least in part on the first administration-related data and the second administration-related data. At Step 1050, the second fluid administration signal can be sent to the flow controller. The physiological processes of a subject can provide, or be used to calculate, one or more parameters such as of cardiac output, stroke volume, etc. The mass flow (rate) parameter can include total volume or rate.

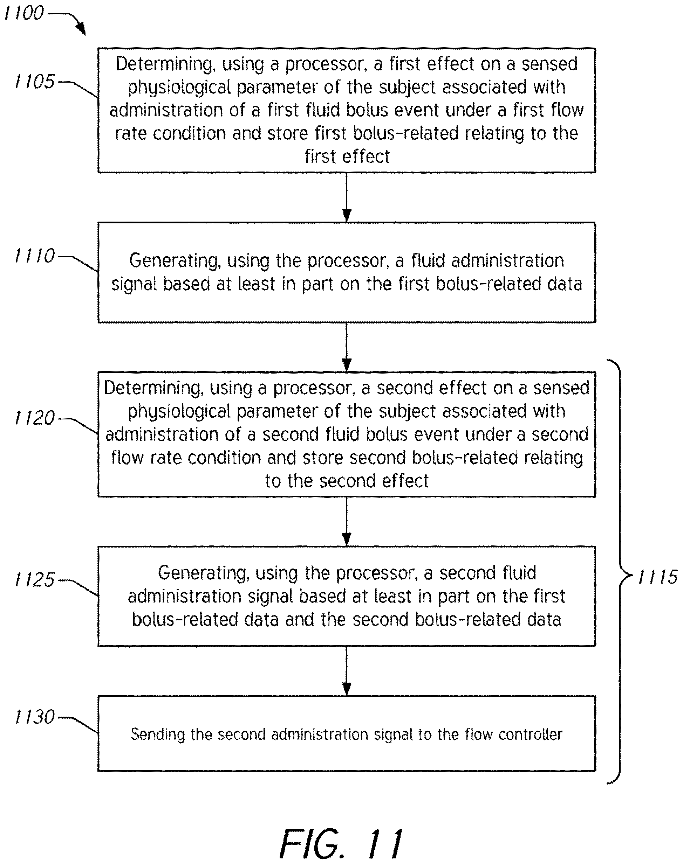

FIG. 11 illustrates a flowchart of an example process 1100 for providing assisted fluid delivery management in accordance with the systems, apparatuses, and methods disclosed herein. The process 1100 can be performed by any of the subject monitoring systems 100 illustrated in FIGS. 1A-1F and FIG. 2 and/or the systems 100a, 100b, 100c, 100d of FIGS. 3A, 3B, 4A, and 4B. Accordingly, for ease of description, the process 1100 is described as being performed by the system and/or a processor of the system. However, it is to be understood that the process 1100 can be performed by any component or combination of components of the systems described herein. Similarly, any step, portion of a step, or combination of steps in the process 1100 can be performed by any component or combination of components of the systems described herein. The process 1100 is an example and not intended to limit the scope of the disclosure. The process 1100 can be altered, e.g., by having steps added, removed, rearranged, combined and/or performed concurrently.

The process 1100 starts at step 1105 where the processor, e.g., a processor module, determines a first effect on the sensed physiological parameter of the subject associated with administration to the subject of a first fluid bolus event under a first flow rate condition, and then stores, using the processor first bolus related data relating to the first effect corresponding to the first flow rate condition. The first effect is determined by analyzing signals received from a physiological sensor and a flow probe and can include additional external and internal physiological information relating to the patient's physiological status, or the additional external information can be subject-population data, patient-specific or historical information stored in a computer readable storage medium or in accessible memory. The first bolus-related data relating to the first effect can be stored in accessible memory, such as RAM, ROM, flash or other type of computer readable storage medium. At step 1110, the processor generates a fluid administration signal based at least in part upon the first bolus-related data where the fluid administration signal is usable to assist in administration fluid to the subject. In some embodiments, the processor sends the first fluid administration signal to a flow controller, the flow controller configured to control an amount of infusion fluid administered to a subject

In some embodiments, process 1100 can further provide additional processing steps 1115. For example, at step 1120, the processor determines a second effect on the sensed physiological parameter of the subject associated with an administration to the subject of a second fluid bolus event corresponding to a second flow rate condition and then stores, using the processor, second bolus-related data relating to the second effect corresponding to the second flow rate condition. In some embodiments, the second fluid bolus corresponds to infusion of a fluid to the subject at an infusion rate that is the same or different from the first fluid bolus. At step 1125, the processor generates a second fluid administration signal based at least in part upon the first administration-related data and the second administration-related data, where the second fluid administration signal is usable to assist in administration of fluid to the subject. At step 1130, the processor sends the second fluid administration signal to the flow controller.

In some embodiments, the processor and an algorithm are configured to generate a representation of an intervention parameter on a user interface corresponding to the first and/or second fluid administration signals. A user interface can be configured to receive user input indicating acceptance or rejection of the intervention parameter. Responsive to an acceptance of the intervention parameter, the processor can generate a control signal for the flow controller. The algorithm can be used to determine one or more intervention commands and present such commands, e.g., as options to the user on a monitor or its display. The intervention commands can include, for example, the type of fluid bolus to administer, an amount of fluid bolus to administer, a rate and time over which to administer the fluid bolus, and/or a start/stop option.

The methods described herein are configured for assisted delivery hemodynamic management in combination with the systems and apparatuses disclosed herein. Accordingly, the disclosed methods can be performed by any of the systems 100 described herein with reference to FIGS. 1A-1F and 2 and the systems 100a-d described herein with reference to FIGS. 3A, 3B, 4A, and 4B. For example, the processing module 541 (described herein with reference to FIG. 2) can receive administration-related data relating to one or more physiological parameters of the subject. The administration-related data can be received from one or more of the physiological or HD sensor 204 and flow probe 119 or from user input received through interaction with the monitor 310 by way of the graphics engine 549. Upon receiving the administration-related data, the processing module 541 stores administration-related information corresponding to the administration-related data in in accessible memory, such as RAM, ROM, flash or other type of computer readable storage medium. The algorithm 107, using one or more of subject-population data, patient specific data, and/or historic data and the administration-related data, can determine, based at least in part upon the administration-related data, a predicted change in a physiological parameter of the subject 120 in response to administration of a fluid bolus to the subject. The algorithm 107 provides a fluid administration recommendation in response to the predicted change to the graphics engine 549 or monitor 310 on a display or user interface. The user can then respond with a response relating to the recommendation from the algorithm 107. The algorithm 107 can receive user input relating to a recommendation and generate an administration signal related to the recommendation that is sent to the flow controller 301. Alternatively, the fluid administration recommendation can be converted to a signal to cause the fluid flow controller 301 to administer the recommendation automatically.

Another example method for assisted delivery hemodynamic management now described can be used in combination with the systems and apparatuses disclosed herein. For example, the method can be performed by the systems 100 described herein with reference to FIGS. 1A-1F and 2 including the systems 100a-d described herein with reference to FIGS. 3A, 3B, 4A, and 4B. The method can include using a processing module for determining a first effect on a physiological parameter of the subject associated with administration to the subject of a first fluid bolus corresponding to a first mass flow parameter and storing a first administration-related data relating to the first effect in accessible memory, such as RAM, ROM, flash or other type of computer readable storage medium. In some embodiments, the first effect corresponds to providing an infusion of a fluid to the subject at a rate of about 2 L/hour to about 6 L/hour. The processing module can then determine a second effect on a physiological parameter of the subject associated with administration to the subject of a second fluid bolus corresponding to a second mass flow or fluid flow rate parameter, and store second administration-related data relating to the second effect in accessible memory, such as RAM, ROM, flash or other type of computer readable storage medium. In some embodiments, the second fluid bolus corresponds to infusion of a fluid to the subject at an infusion rate that is the same or different from the first fluid bolus. A fluid administration signal and/or a fluid administration recommendation can then be determined and provided based at least in part upon at least one of the first effect and the second effect in combination with one or more of subject-population data, historical data, and patient-specific data. The recommendation can be provided to the user on a user interface for acceptance or rejection of the recommendation. In some embodiments, the fluid administration recommendation can be converted to a signal to cause the fluid flow controller to administer the recommendation automatically.

Any infusion fluid known in the art can be used in conjunction with the present system and the disclosed physiological sensors. The IV solution can contain a medicament, such as but not limited to heparin and/or vasodilators and/or cardiac-related medications.

In some embodiments, one, two, or more solutions can be used in conjunction with the disclosed systems. For example, in some embodiments, two or more solutions can be used or administered to the subject concurrently, intermittently, or independently. In some embodiments, the infusion fluid is an isotonic saline solution containing a sufficient concentration of an anticoagulant to substantially prevent blood clotting in and/or near a catheter (not shown) and/or a physiological sensor, and/or a sufficient concentration of or antimicrobial to substantially prevent infection in and/or near the catheter or other insertion device (not shown).

It is generally known that, for a variety of reasons, there may be a delay in correlation between a hemodynamic parameter acquisition event and that of a bolus infusion, e.g., the measured mass flow amount or rate of infusate corresponding to the onset, peak, and or decay profile of a hemodynamic response. The system is configurable to compensate and/or adjust for the "lag" of the flow probe data with that of the sensed hemodynamic data using correction factors empirically or experimentally determined, or historic data.

### Examples of Flushing Fluid Monitoring Devices

Tracking and/or controlling fluid balance during patient care and/or surgery may result in advantageous clinical results. Ideally, all fluids that go in and out of the patient should be monitored to maintain a fluid balance around zero. For example, under circumstances where there is bleeding out, fluids such as flushing fluids are generally introduced to maintain blood pressure among other things. Under such circumstances, the flushing fluid is mixed with lost blood. It is advantageous to track blood loss by determining the amount of blood that is mixed with the flushing fluid. Accordingly, disclosed herein are devices configured to measure and track the amount of flushing fluid that is administered during a procedure. The apparatuses, systems, and methods disclosed herein can be used to determine or monitor fluid volumes that are administered to a subject. This information can be used to determine the amount of flushing fluid delivered to a patient. Using this information, the amount of blood lost can be calculated or determined from the mixture of blood and flushing fluid that is collected during the procedure.

The disclosed flushing devices and associated methods are configured to monitor the usage of flushing liquid during an operation or other similar procedure. Accurately determining the amount of flushing liquid can advantageously lead to an accurate determination of the amount of blood loss in a patient. This can help clinicians, doctors, and other healthcare providers to assess the status of the patient more confidently and accurately. The disclosed devices and methods use a flow sensor configured to measure the flow rate or the amount of liquid flow through the flushing device. The flow sensor is similar to the flow probes/flow sensors 119 described herein with respect to the system 100 of FIGS. 1A-1F, 2 and the systems 100a-100d of FIGS. 3A, 3B, 4A, and 4B.

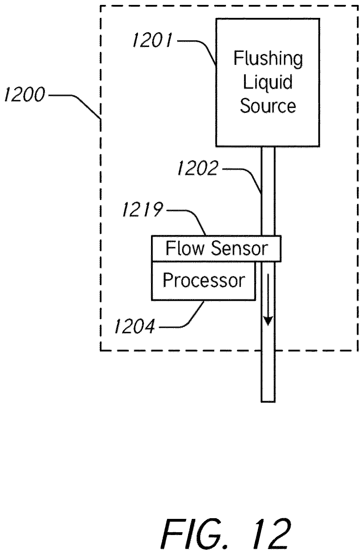

FIG. 12 illustrates an example flushing device 1200 that includes a flow sensor 1219 configured to measure a flow rate of liquid from the device 1200. The flushing device 1200 includes a conduit 1202 configured to deliver flushing liquid from a flushing liquid source 1201 to a surgical site. The flushing device 1200 also includes a processor 1204 communicatively coupled to the flow sensor 1219. The processor 1204 is configured to determine a volume of flushing liquid delivered by the flushing device 1200.

The flow sensor 1219 is coupled to the conduit 1202 so that the flow sensor 1219 can measure a flow rate and/or other flow-related data of the flushing liquid flowing through the conduit 1202. The flow sensor 1219 can be positioned within the conduit, partially within the conduit, or external to the conduit. The flow sensor 1219 can be an ultrasonic-based measurement device, a thermal-based measurement device, a pressure-based measurement device, a Venturi-based measurement device, or a combination of measurement technologies. The flow sensor 1219 can be any suitable flow sensor for measuring flow rates of a liquid. The flow sensor 1219 can be a disposable sensor. The disposable version of the flow sensor 1219 can include or exclude the processor 1204.

The flow sensor 1219 is communicatively coupled to the processor 1204 so that the processor receives signals generated by the flow sensor 1219. The processor 1204 can be housed and/or integrated with the flow sensor, forming an integrated device.

The processor 1204 is configured to receive flow-related signals from the flow sensor 1219 and to determine a flow rate, flow volumes, changes in flow rates, based on the received flow-related signals. The processor 1204 is configured to utilize one or more algorithms to transform the received flow-related signals to measurements of the flow rate, flow volume.

The processor 1204 can be integrated with the flushing device 1200, separate from the flow sensor 1219. In such embodiments, the processor 1204 can be connected to the flow sensor 1219 when the flow sensor is attached or coupled to the conduit 1202. In certain implementations, the flow sensor 1219 is a disposable that is configured to be releasably coupled to the conduit 1202. In such implementations, coupling the flow sensor 1219 to the conduit 1202 can establish sufficient electrical connection between the processor 1204 and the flow sensor 1219 so that the processor receives the flow-related signals from the flow sensor 1219. In some embodiments, the flow sensor 1219 communicates wirelessly with the processor 1204. In some embodiments, the processor 1204 is a separate device from the flow sensor 1219 and can be a separate computing device. The processor 1204 can include a memory configured to execute instructions that determine the volume of liquid delivered to a patient based at least in part on measurements provided by the flow sensor 1219.

The flushing liquid source 1201 includes one or more containers containing infusion liquid, for example a number of bags containing saline fluid, such as NaCl 0.9%. The one or more containers of the flushing liquid source 1201 are connected to a pump or other device configured to pump infusion liquid to the conduit 1202 for delivery to a surgical site. The pump device may be any suitable pump device for infusion liquid. In some embodiments, the flushing liquid source 1201 includes a flow control apparatus configured to increase, decrease, start, and/or stop the flow of flushing liquid out of the flushing liquid source 1201.

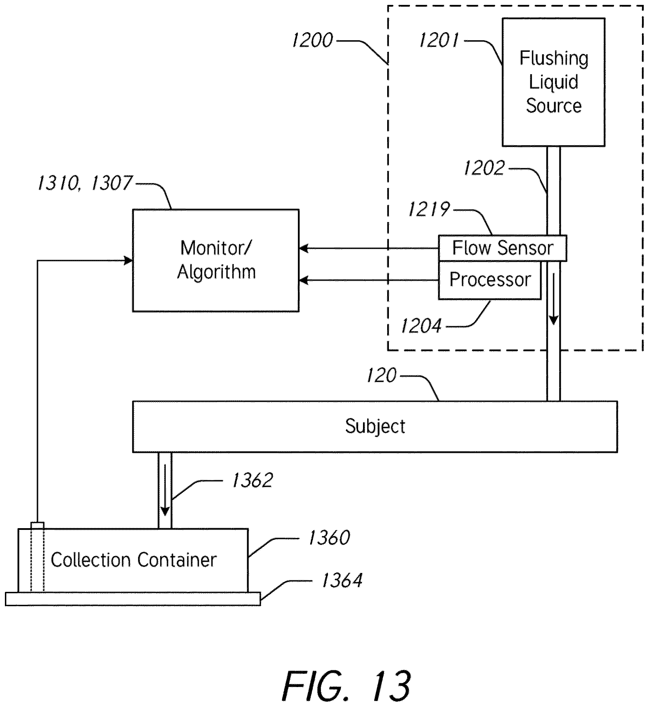

FIG. 13 illustrates a monitoring system 1300 that includes the flushing device 1200 of FIG. 12, a monitor 1310 with algorithm 1307, and a collection container 1360 configured to collect flushing liquid and blood at a surgical site. The monitor 1310 is similar to the monitor 310 described herein with reference to FIGS. 1A-1F, 2, 3A, 3B, 4A, and 4B. Furthermore, the algorithm 1307 is similar to the algorithm 107 described herein with reference to FIGS. 1A-1F, 2, 3A, 3B, 4A, and 4B. The algorithm 1307 is configured to receive flow sensor data and to determine flow-related data from the flow sensor 1219 and/or the processor 1204, the flow-related data including, for example, flow rate, flow volume, total liquid flow, changes in flow rate. The monitor 1310 can be configured to display flow-related data received from the flow sensor 1219, the processor 1204, and/or determined by the algorithm 1307.

The flow sensor 1219 can be clamped on the conduit 1202 and/or integrated to the conduit 1202 of the flushing device 1200. The flow sensor 1219 is configured to detect the flow of liquid through the conduit 1202. The flow sensor 1219, the processor 1204, and/or the algorithm 1307 is configured to determine the flow rate or other flow-related data to determine the amount of flushing liquid used. This information can be displayed on the monitor 1310, on a sensor housing of the flow sensor 1219, or it can be sent to a hospital information system using a wired or wireless connection.

The monitoring system 1300 also includes a collection container 1360 fluidically coupled to the surgical site (e.g., the subject 120) through an effusion conduit 1362. The effusion conduit 1362 is configured to carry effusion liquid from the subject to the collection container 1360. The effusion liquid includes a mixture of blood and infusion liquid from the flushing liquid device 1200.

The monitoring system 1300 can include a collection container sensor 1364 configured to provide signals indicative of a total volume of liquid in the collection container 1360. The collection container sensor 1364 can be configured to generate signals based on a volume and/or weight of liquid in the collection container 1360. The collection container sensor 1364 can be communicably coupled to the monitor 1310 and/or the algorithm 1307 to provide liquid volume signals indicative of the volume of liquid in the collection container 1360. In some embodiments, the collection container sensor 1364 is configured to determine the volume of liquid in the collection container 1360 using one or more processors, memory, and computer executable instructions that related measured data to liquid volumes. In some embodiments, the collection container sensor 1364 transmits volume-related signals (e.g., either wirelessly or through wired communication) to the monitor 1310 and/or algorithm 1307 that in turn determines the volume of liquid in the collection container 1360.

The algorithm 1307 is configured to receive signals indicative of a flow rate and/or flow volume of the flushing device 1200 and signals indicative of the volume of liquid in the collection container 1360 and to determine the amount of blood lost from the subject 120 based on the received signals. In some embodiments, the amount of blood lost is determined by finding a difference between the volume of liquid in the collection container 1360 and the volume of liquid delivered by the flushing device 1200. In some embodiments, the algorithm 1307 can estimate corrections to this difference based on tissue properties, absorption of flushing liquid, and/or other sources of uncertainty or liquid loss.

The monitor 1310 can be configured to display the determined amount of blood loss. The monitor 1310 can include a processor and a memory configured to execute instructions that determine the volume of liquid delivered to the subject 120 based at least in part on measurements provided by the flow sensor 1219 and that determine the volume of liquid in the collection container 1360 based at least in part on measurements provided by the collection container sensor 1364.

### Example Methods of Determining Blood Loss

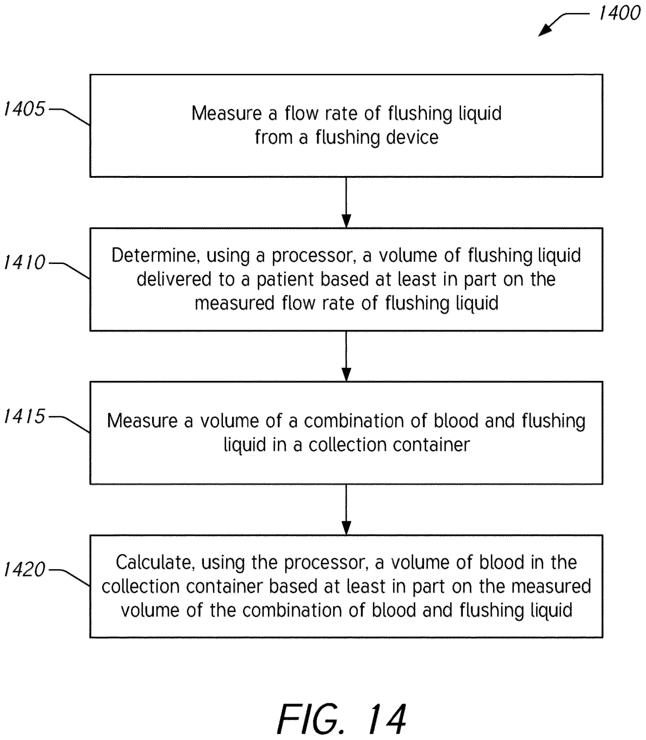

FIG. 14 illustrates a flow chart of an example method 1400 for determining the amount of blood lost from a patient during a procedure. The method 1400, which is not claimed, can be performed using any of the flushing devices and/or monitoring systems described herein, such as the devices and systems described herein with reference to FIGS. 12 and 13. The method 1400 is advantageous because it tracks the amount of flushing fluid delivered to the patient, measures the amount of fluid collected from the surgical site, and determines the amount of blood and flushing liquid collected from the surgical site. This provides an accurate determination of the amount of blood loss.

The method 1400 can be performed by any of the devices or systems described herein. For ease of description, the method 1400 is described as being performed by the monitoring system 1300 of FIG. 13. However, it is to be understood that the subject monitoring systems 100 of FIGS. 1A-1F and 2, the systems 100a-100d of FIGS. 3A, 3B, 4A, and 4B, the flushing device 1200 described herein with reference to FIG. 12, and/or the monitoring system 1300 described herein with reference to FIG. 13 can perform the method 1400. It is also to be understood that any component, subsystem, device, or apparatus of the subject monitoring system 100 of FIGS. 1A-1F and 2, the systems 100a-100d of FIGS. 3A, 3B, 4A, and 4B, the flushing device 1200, and/or the monitoring system 1300 can perform any portion of a step, any individual step, or any combination of steps in the method 1400. Similarly, any combination of components, subsystems, devices, or apparatuses of the subject monitoring system 100 of FIGS. 1A-1F and 2, the systems 100a-100d of FIGS. 3A, 3B, 4A, and 4B, the flushing device 1200, and/or the monitoring system 1300 can perform any portion of a step, any individual step, or any combination of steps in the method 1400.

In step 1405, the monitoring system measures a flow rate of flushing liquid from a flushing device. In step 1410, the monitoring system determines a volume of flushing liquid delivered to a patient. In step 1415, the monitoring system measures a volume of a combination of blood and flushing liquid in a collection container. In step 1420, the monitoring system calculates a volume of blood in the collection container.

### Additional Embodiments

In any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

Conditional language used herein, such as, among others, "can," "could," "might," "may," "e.g.,", unless specifically stated otherwise, or otherwise understood within the context as used, is intended in its ordinary sense and is generally intended to convey that certain embodiments include, while other embodiments do not include, certain features, elements and/or steps. Thus, such conditional language is not generally intended to imply that features, elements and/or steps are in any way required for one or more embodiments. The terms "comprising," "including," "having," "characterized by," are synonymous, are used in their ordinary sense, and are used inclusively, in an open-ended fashion, and do not exclude additional elements, features, acts, operations. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain embodiments require at least one of X, at least one of Y and at least one of Z to each be present.

Reference throughout this specification to "certain embodiments" or "an embodiment" means that a particular feature, structure or characteristic described in connection with the embodiment is included in at least some embodiments. Thus, appearances of the phrases "in some embodiments" or "in an embodiment" in various places throughout this specification are not necessarily all referring to the same embodiment and may refer to one or more of the same or different embodiments. Furthermore, the particular features, structures or characteristics can be combined in any suitable manner, as would be apparent to one of ordinary skill in the art from this disclosure, in one or more embodiments.

It should be appreciated that in the above description of embodiments, various features are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular embodiment herein can be applied to or used with any other embodiment(s).

## Claims

1. A subject monitoring system (100), comprising
a fluid source (321) for a fluid to be administered to a subject;
a flow controller (301) associated with the fluid source and configured to vary a fluid administration rate and a volume of the fluid to be administered;
a flow probe (119), which is configured to sense flow-related information related to an administration of the fluid to the subject and to provide flow-related data indicative of a fluid flow rate related to the administration of the fluid to the subject (120);
a physiological sensor (204), which is configured to detect and quantify a physiological parameter of the subject and to provide physiological data indicative thereof;
a monitor (310, 107) including a processor (542) and a display, for executing an algorithm (107, 800) that includes
receiving (805) the physiological data provided by the physiological sensor (204) and correlated flow-related data provided by the flow probe (119),
determining (810) administration-related data indicative of a response of the subject to administered volumes and rates of fluid administration, and
based upon the administration-related data, generating and providing (815) via the display a recommendation for a subsequent administration protocol controlling the flow controller, the subsequent administration protocol including an amount or a rate of fluid to be administered to the subject.

2. The system of claim 1, wherein the flow controller (301) is manually controllable.

3. The system of claim 1, wherein the flow controller (301) is configured to be controlled automatically, and wherein the monitor (310, 107) is configured, in executing the algorithm, to control the flow controller (301) to vary a fluid administration rate in accordance with the subsequent administration protocol, and wherein the system is configured to allow a manual override of the subsequent administration protocol by a clinician.

4. The system of claim 1, wherein the physiological sensor is a hemodynamic sensor (204).

5. The system of claim 4, wherein the hemodynamic sensor (204) is configured to provide a parameter directly or indirectly related to providing or calculating stroke volume, cardiac output, end-diastolic volume, ejection fraction, stroke volume variation, pulse pressure variation, systolic pressure variations, extravascular lung water index, pulmonary vascular permeability index, global end-diastolic index, global ejection fraction, systolic volume index, arterial blood pressure, cardiac index, systemic vascular resistance index, peripheral resistance, central venous saturation, and plethysmographic variability index.

6. The system of claim 1, wherein the processor (542) is configured, in executing the algorithm,
to determine a first effect on a physiological state of the subject associated with the received flow-related data, which corresponds to an amount of fluid administered to the subject,
to store first administration-related data relating to the first effect within a device storage, and
to generate the recommendation for the subsequent administration protocol based upon the first administration related data.

7. The system of claim 1, wherein the processor (542) is configured to calculate a predicted change in the subject's physiological parameter in response to administration of a first or second fluid bolus to the subject, using a process based at least in part on one or more of a subject-population database or a patient history record.

8. The system of claim 1, wherein the fluid source (321) is a fluid delivery device with an infusion rate of about 6 liter/hour, 5 liter/hour, 4 liter/hour, 3 liter/hour, or 2 liter/hour.

9. The system of claim 1, comprising a device (1300) for monitoring an amount of blood lost from a subject (120), the device comprising:
a flushing liquid device (1200) comprising the fluid source in the form of a flushing liquid source (1201), a conduit (1202) configured to direct flushing liquid from the flushing liquid source to the subject (120), and the flow probe in the form of a flow sensor (1219) coupled to the conduit, the flow sensor configured to provide flow-related signals corresponding to a flow of the flushing liquid through the conduit;
a collection container (1360) configured to receive effusion liquid from the subject, the effusion liquid including blood and flushing liquid;
a collection container sensor (1364) configured to provide volume-related signals corresponding to a volume of the effusion liquid in the collection container (1360); and
the monitor (1310) is configured to receive the flow-related signals from the flow sensor (1219), to receive the volume-related signals from the collection container sensor (1364), and to determine a volume of blood in the collection container (1360).

10. The system of claim 9, wherein the monitor (1310) is configured to determine a volume of flushing liquid delivered to the subject (120) using the flow-related signals provided by the flow sensor (1219), and to determine the amount of blood lost by finding a difference between the determined volume of liquid in the collection container and the determined volume of flushing liquid delivered by the flushing liquid device (1200).

11. The system of claim 9, wherein the flushing liquid device (1200) further comprises a processor (1204) coupled to the flow sensor (1219), the processor configured to execute computer-executable instructions to derive a volume of flushing liquid delivered to the subject (120) based at least in part on the flow-related signals provided by the flow sensor (1219).

12. The system of claim 11, wherein the processor is configured to provide flushing liquid volume signals to the monitor (1310).

13. The system of claim 9, wherein the collection container sensor (1364) is configured to provide signals indicative of a weight of liquid in the collection container (1360).

14. The system of claim 9, further comprising an effusion pump configured to direct the effusion liquid from the subject to the collection container.

15. The system of claim 9, wherein the flushing liquid device further comprises a pump configured to direct the flushing liquid from the flushing liquid source (1201) to the conduit (1202) or an effusion pump configured to direct the effusion liquid from the subject (120) to the collection container (1360).

## Patentansprüche

1. Subjektüberwachungssystem (100), umfassend:
eine Fluidquelle (321) für ein Fluid, das einem Subjekt verabreicht werden soll;
einen Durchflussregler (301), der mit der Fluidquelle assoziiert ist und ausgestaltet ist, um eine Fluidverabreichungsrate und ein Volumen des zu verabreichenden Fluids zu variieren;
eine Durchflusssonde (119), die ausgestaltet ist, um mit Durchfluss zusammenhängende Informationen, die mit einer Verabreichung des Fluids an das Subjekt in Beziehung stehen, abzufühlen und mit Durchfluss zusammenhängende Daten bereitzustellen, die eine Fluiddurchflussrate angeben, die mit der Verabreichung des Fluids an das Subjekt (120) in Beziehung steht;
einen physiologischen Sensor (204), der ausgestaltet ist, um einen physiologischen Parameter des Subjekts zu detektieren und zu quantifizieren, und um physiologische Daten bereitzustellen, die Angaben dazu liefern;
einen Monitor (310, 107), der einen Prozessor (542) und eine Anzeige einschließt, um einen Algorithmus (107, 800) auszuführen, der einschließt:
Empfangen (805) von den physiologischen Daten, die von dem physiologischen Sensor (204) bereitgestellt werden, und den korrelierten, mit Durchfluss zusammenhängenden Daten, die von der Durchflusssonde (119) bereitgestellt werden,
Bestimmen (810) von mit Verabreichung zusammenhängenden Daten, die eine Reaktion des Subjekts auf verabreichte Volumina und Raten der Fluidverabreichung angeben, und
basierend auf den mit Verabreichung zusammenhängenden Daten Generieren und Bereitstellen (815) einer Empfehlung für ein nachfolgendes Verabreichungsprotokoll, das den Durchflussregler steuert, mittels der Anzeige, wobei das nachfolgende Verabreichungsprotokoll eine Menge oder eine Rate von an das Subjekt zu verabreichendem Fluid einschließt.

2. System nach Anspruch 1, wobei der Durchflussregler (301) manuell steuerbar ist.

3. System nach Anspruch 1, wobei der Durchflussregler (301) ausgestaltet ist, um automatisch gesteuert zu werden, und wobei der Monitor (310, 107) ausgestaltet ist, um beim Ausführen des Algorithmus den Durchflussregler (301) zu steuern, um eine Fluidverabreichungsrate gemäß dem nachfolgenden Verabreichungsprotokoll zu variieren, und wobei das System ausgestaltet ist, um manuelles Überschreiben des nachfolgenden Verabreichungsprotokolls durch einen Kliniker zuzulassen.

4. System nach Anspruch 1, wobei der physiologische Sensor ein hämodynamischer Sensor (204) ist.

5. System nach Anspruch 4, wobei der hämodynamische Sensor (204) ausgestaltet ist, um einen Parameter bereitzustellen, der direkt oder indirekt mit dem Bereitstellen oder Berechnen von Schlagvolumen, Herzzeitvolumen, enddiastolischem Volumen, Auswurffraktion, Schlagvolumenvariation, Pulsdruckvariation, systolischen Druckvariationen, extravaskulärem Lungenwasserindex, pulmonarem vaskulärem Permeabilitätsindex, globalem enddiastolischem Index, globaler Auswurffraktion, systolischem Volumenindex, arteriellem Blutdruck, Herzindex, systemischem vaskulärem Widerstandsindex, peripherem Widerstand, zentralvenöser Sättigung und plethysmographischem Variabilitätsindex in Beziehung steht.

6. System nach Anspruch 1, wobei der Prozessor (542) ausgestaltet ist, um bei der Ausführung des Algorithmus folgendes zu tun:
Bestimmen eines ersten Effekts auf einen physiologischen Zustand des Subjekts, der mit den empfangenen mit Durchfluss zusammenhängenden Daten assoziiert ist, die einer Menge an dem Subjekt verabreichtem Fluid entsprechen,
Speichern von ersten mit Verabreichung zusammenhängenden Daten, die mit dem ersten Effekt in Beziehung stehen, im Speicher einer Vorrichtung, und
Generieren der Empfehlung für das nachfolgende Verabreichungsprotokoll basierend auf den ersten mit Verabreichung zusammenhängenden Daten.

7. System nach Anspruch 1, wobei der Prozessor (542) ausgestaltet ist, um eine vorhergesagte Änderung im physiologischen Parameter des Subjekts in Reaktion auf die Verabreichung eines ersten oder zweiten Fluidbolus an das Subjekt unter Verwendung eines Prozesses zu berechnen, der mindestens teilweise auf einem oder mehreren von einer Subjektpopulationsdatenbank oder einer Patientenhistorienaufzeichnung basiert.

8. System nach Anspruch 1, wobei die Fluidquelle (321) eine Fluidzuführungsvorrichtung mit einer Infusionsrate von etwa 6 Liter/Stunde, 5 Liter/Stunde, 4 Liter/Stunde, 3 Liter/Stunde oder 2 Liter/Stunde ist.

9. System nach Anspruch 1, umfassend eine Vorrichtung (1300) zum Überwachen einer Menge an Blut, die ein Subjekt (120) verloren hat, wobei die Vorrichtung umfasst:
eine Spülflüssigkeitsvorrichtung (1200), die die Fluidquelle in Form einer Spülflüssigkeitsquelle (1201), eine Leitung (1202), die ausgestaltet ist, um Spülflüssigkeit aus der Spülflüssigkeitsquelle zu dem Subjekt (120) zu lenken, und die Durchflusssonde in Form eines Durchflusssensors (1219), der an die Leitung gekoppelt ist, umfasst, wobei der Durchflusssensor ausgestaltet ist, um mit Durchfluss zusammenhängende Signale bereitzustellen, die einem Durchfluss der Spülflüssigkeit durch die Leitung entsprechen;
einen Sammelbehälter (1360), der ausgestaltet ist, um Effusionsflüssigkeit von dem Subjekt zu empfangen, wobei die Effusionsflüssigkeit Blut und Spülflüssigkeit einschließt;
einen Sammelbehältersensor (1364), der ausgestaltet ist, um mit Volumen zusammenhängende Signale bereitzustellen, die einem Volumen der Effusionsflüssigkeit in dem Sammelbehälter (1360) entsprechen; und
wobei der Monitor (1310) ausgestaltet ist, um die mit Durchfluss zusammenhängenden Signale von dem Durchflusssensor (1219) zu empfangen, die mit Volumen zusammenhängenden Signale von dem Sammelbehältersensor (1364) zu empfangen, und ein Volumen von Blut in dem Sammelbehälter (1360) zu bestimmen.

10. System nach Anspruch 9, wobei der Monitor (1310) ausgestaltet ist, um ein Volumen an Spülflüssigkeit, das dem Subjekt (120) zugeführt wurde, unter Verwendung der mit Durchfluss zusammenhängenden Signale, die von dem Durchflusssensor (1219) bereitgestellt wurden, zu bestimmen, und die Menge an verlorenem Blut zu bestimmen, indem eine Differenz zwischen dem bestimmten Volumen an Flüssigkeit in dem Sammelbehälter und dem bestimmten Volumen an Spülflüssigkeit, das durch die Spülflüssigkeitsvorrichtung (1200) zugeführt wurde, gefunden wird.

11. System nach Anspruch 9, wobei die Spülflüssigkeitsvorrichtung (1200) des Weiteren einen Prozessor (1204) umfasst, der an den Durchflusssensor (1219) gekoppelt ist, wobei der Prozessor ausgestaltet ist, um computerausführbare Anweisungen auszuführen, um ein Volumen an Spülflüssigkeit, welches dem Subjekt (120) zugeführt wurde, mindestens teilweise basierend auf den mit Durchfluss zusammenhängenden Signalen, die durch den Durchflusssensor (1219) bereitgestellt wurden, abzuleiten.

12. System nach Anspruch 11, wobei der Prozessor ausgestaltet ist, um dem Monitor (1310) Spülflüssigkeitsvolumensignale bereitzustellen.

13. System nach Anspruch 9, wobei der Sammelbehältersensor (1364) ausgestaltet ist, um Signale bereitzustellen, die ein Gewicht der Flüssigkeit in dem Sammelbehälter (1360) angeben.

14. System nach Anspruch 9, des Weiteren umfassend eine Effusionspumpe, die ausgestaltet ist, um die Effusionsflüssigkeit von dem Subjekt zu dem Sammelbehälter zu lenken.

15. System nach Anspruch 9, wobei die Spülflüssigkeitsvorrichtung des Weiteren eine Pumpe, die ausgestaltet ist, um die Spülflüssigkeit aus der Spülflüssigkeitsquelle (1201) zu der Leitung (1202) zu lenken, oder eine Effusionspumpe umfasst, die ausgestaltet ist, um die Effusionsflüssigkeit von dem Subjekt (120) zu dem Sammelbehälter (1360) zu lenken.

## Revendications

1. Système de surveillance (100) d'un sujet, comprenant
une source de fluide (321) destinée à un fluide à administrer à un sujet ;
un dispositif de commande (301) d'écoulement associé à la source de fluide et conçu pour faire varier un débit d'administration de fluide et un volume du fluide à administrer ;
une sonde (119) d'écoulement, qui est conçue pour détecter des informations liées à l'écoulement liées à une administration du fluide au sujet et pour fournir des données liées à l'écoulement indicatives d'un débit d'écoulement de fluide lié à l'administration du fluide au sujet (120) ;
un capteur physiologique (204), qui est conçu pour détecter et quantifier un paramètre physiologique du sujet et pour fournir des données physiologiques indicatives de celui-ci ;
un moniteur (310, 107) comprenant un processeur (542) et un affichage, destiné à exécuter un algorithme (107, 800) comprenant
la réception (805) des données physiologiques fournies par le capteur physiologique (204) et des données liées à l'écoulement corrélées fournies par la sonde (119) d'écoulement,
la détermination (810) de données liées à l'administration indicatives d'une réponse du sujet à des volumes et des débits d'administration de fluide administrés et
sur la base des données liées à l'administration, la génération et la fourniture (815) par l'intermédiaire de l'affichage d'une recommandation pour un protocole d'administration ultérieur commandant le dispositif de commande d'écoulement, le protocole d'administration ultérieur comprenant une quantité ou un débit de fluide à administrer au sujet.

2. Système selon la revendication 1, le dispositif de commande (301) d'écoulement pouvant être commandé manuellement.

3. Système selon la revendication 1, le dispositif de commande (301) d'écoulement étant conçu pour être commandé automatiquement et le moniteur (310, 107) étant conçu, lors de l'exécution de l'algorithme, pour commander le dispositif de commande (301) d'écoulement afin de faire varier un débit d'administration de fluide conformément au protocole d'administration ultérieur et
le système étant conçu pour permettre un surpassement manuel du protocole d'administration ultérieur par un clinicien.

4. Système selon la revendication 1, le capteur physiologique étant un capteur hémodynamique (204).

5. Système selon la revendication 4, le capteur hémodynamique (204) étant conçu pour fournir un paramètre lié directement ou indirectement à la fourniture ou au calcul d'un volume systolique, d'un débit cardiaque, d'un volume diastolique final, d'une fraction d'éjection, d'une variation de volume systolique, d'une variation de pression du pouls, de variations de pression systolique, d'un indice d'eau pulmonaire extravasculaire, d'un indice de perméabilité vasculaire pulmonaire, d'un indice diastolique final global, d'une fraction d'éjection globale, d'un indice de volume systolique, d'une tension artérielle, d'un indice cardiaque, d'un indice de résistance vasculaire systémique, d'une résistance périphérique, d'une saturation veineuse centrale et d'un indice de variabilité pléthysmographique.

6. Système selon la revendication 1, le processeur (542) étant conçu, lors de l'exécution de l'algorithme,
pour déterminer un premier effet sur un état physiologique du sujet associé aux données liées à l'écoulement reçues, qui correspond à une quantité de fluide administrée au sujet,
pour stocker des premières données liées à l'administration relatives au premier effet à l'intérieur d'un dispositif de stockage de dispositif et
pour générer la recommandation pour le protocole d'administration ultérieur sur la base des premières données liées à l'administration.

7. Système selon la revendication 1, le processeur (542) étant conçu pour calculer un changement prédit du paramètre physiologique du sujet en réponse à l'administration d'un premier ou d'un second bolus de fluide au sujet, à l'aide d'un procédé basé au moins en partie sur un ou plusieurs éléments parmi une base de données de population de sujets ou un enregistrement d'antécédents de patient.

8. Système selon la revendication 1, la source de fluide (321) étant un dispositif d'administration de fluide présentant un débit de perfusion d'environ 6 litres/heure, 5 litres/heure, 4 litres/heure, 3 litres/heure, ou 2 litres/heure.

9. Système selon la revendication 1, comprenant un dispositif (1300) destiné à surveiller une quantité de sang perdue d'un sujet (120), le dispositif comprenant :
un dispositif à liquide de rinçage (1200) comprenant la source de fluide sous la forme d'une source de liquide de rinçage (1201), un conduit (1202) conçu pour diriger le liquide de rinçage à partir de la source de liquide de rinçage au sujet (120) et la sonde d'écoulement sous la forme d'un capteur d'écoulement (1219) accouplé au conduit, le capteur d'écoulement étant conçu pour fournir des signaux liés à l'écoulement correspondant à un écoulement du liquide de rinçage à travers le conduit ;
un récipient de collecte (1360) conçu pour recevoir un liquide d'épanchement provenant du sujet, le liquide d'épanchement comprenant du sang et du liquide de rinçage ;
un capteur (1364) de récipient de collecte conçu pour fournir des signaux liés au volume correspondant à un volume du liquide d'épanchement dans le récipient de collecte (1360) ; et
le moniteur (1310) étant conçu pour recevoir les signaux liés à l'écoulement provenant du capteur d'écoulement (1219), pour recevoir les signaux liés au volume provenant du capteur (1364) de récipient de collecte et pour déterminer un volume de sang dans le récipient de collecte (1360).

10. Système selon la revendication 9, le moniteur (1310) étant conçu pour déterminer un volume de liquide de rinçage administré au sujet (120) à l'aide des signaux liés à l'écoulement fournis par le capteur d'écoulement (1219) et pour déterminer la quantité de sang perdue en trouvant une différence entre le volume déterminé de liquide dans le récipient de collecte et le volume déterminé de liquide de rinçage administré par le dispositif à liquide de rinçage (1200).

11. Système selon la revendication 9, le dispositif à liquide de rinçage (1200) comprenant en outre un processeur (1204) accouplé au capteur d'écoulement (1219), le processeur étant conçu pour exécuter des instructions exécutables par ordinateur afin de déduire un volume de liquide de rinçage administré au sujet (120) sur la base, au moins en partie, des signaux liés à l'écoulement fournis par le capteur d'écoulement (1219).

12. Système selon la revendication 11, le processeur étant conçu pour fournir des signaux de volume de liquide de rinçage au moniteur (1310).

13. Système selon la revendication 9, le capteur (1364) de récipient de collecte étant conçu pour fournir des signaux indicatifs d'un poids de liquide dans le récipient de collecte (1360).

14. Système selon la revendication 9, comprenant en outre une pompe d'épanchement conçue pour diriger le liquide d'épanchement à partir du sujet vers le récipient de collecte.

15. Système selon la revendication 9, le dispositif à liquide de rinçage comprenant en outre une pompe conçue pour diriger le liquide de rinçage à partir de la source de liquide de rinçage (1201) vers le conduit (1202) ou une pompe d'épanchement conçue pour diriger le liquide d'épanchement à partir du sujet (120) vers le récipient de collecte (1360).
